# EUROPÄISCHE PATENTANMELDUNG

(11) **EP 1 531 008 A2**
(43) Veröffentlichungstag der Anmeldung: **18.05.2005**
(21) Anmeldenummer: 04105818.1
(22) Anmeldetag: 16.11.2004
(51) Int. Cl.: B05B 11/00

(54) **Kosmetische oder dermatologische Zubereitungen für Spender**

(30) Priorität: 17.11.2003 DE 10354053
(71) Anmelder: Beiersdorf AG, 20245 Hamburg (DE)
(72) Erfinder: LANZENDOERFER, Ghita, 22087, Hamburg (DE); Riedel, Heidi, 22083 Hamburg (DE); RUPPERT, Stephan, 20259, Hamburg (DE); KOHUT, Michaela, 20257, Hamburg (DE); MUNDT, Claudia, 28207, Bremen (DE); ECKERS, Lorenz, 21255, Tostedt (DE); HETZEL, Frank, 21261, Welle (DE); KALLMAYER, Volker, 22525, Hamburg (DE)

(57) **Zusammenfassung**

Kosmetik-Produkte, bestehend aus einem Spender für pastöse Produkte mit einem das pastöse Produkt enthaltenden, im wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet daß die Fördereinrichtung ein zu dem Behälter (1) und dem Kopfstück (3) längsverschiebliches Förderelement (5) umfaßt, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlaßöffnung (53) und eine Förderkanalauslaßöffnung (58) aufweist, die durch eine Verschiebebewegung des Förderelementes (5) relativ zu dem Kopfstück (3) in eine Stellung bringbar ist, in der sich die Förderkanalauslaßöffnung (58) zu dem Ausgabekanal (32) öffnet, und kos metischen oder dermatologischen Zubereitungen, welche mit Hilfe des Spenders appliziert werden können, ohne daß sich dabei die kosmetischen Eigenschaften dieser Zubereitungen fühlbar ändern würden.

## Beschreibung

Die vorliegende Erfindung betrifft kosmetische oder dermatologische Zubereitungen, welche mit Hilfe von Schleppkolbenspendersystemen appliziert werden können, ohne daß sich dabei die kosmetischen Eigenschaften dieser Zubereitungen wesentlich ändern würden.

Spender mit gleitverschieblichen Nachlaufkolben (welche auch als Schleppkolbenspender bezeichnet werden) und handbetätigbare Fördereinrichtungen mit einer volumenveränderlichen Förderkammer sind als Vorratsbehälter in einer Vielzahl von Anwendungsbeispielen bekannt, z. B. für die Körperpflege, in der Medizin für die Applikation von Medikamenten oder auch bei der Bereitstellung pastöser Lebensmittel im Handel. Entsprechend vielgestaltig ist auch die Ausgestaltung der für die Bereitstellung der sehr unterschiedlichen pastösen Massen verwendeten Spender, vor allen in Bezug auf ihren unmittelbaren Förder- und Handhabungsmechanismus.

Derartige Spendersysteme sind selbstverständlich prinzipiell zur Applikation kosmetischer oder dermatologischer Zubereitungen geeignet. Die in einer besonderen Ausführungsform in der WO-03/004374-A1 beschriebenen Schleppkolbenspender mit selbstschließender Öffnung sind dabei insbesondere deswegen vorteilhaft für Kosmetika zu verwenden, da keine Produktreste nach der Applikation aus der Spenderöffnung auslaufen und dadurch eine unästhetische Verschmutzung des Spenders vermieden wird. Andererseits werden die kosmetischen oder dermatologischen Formulierungen bei ihrem Austritt aus dem Spender aufgrund der speziellen Bauart (Dichtungslippe an der Öffnung) einer starken Scherung ausgesetzt, welche einen deutlichen Viskositätsverlust der kosmetischen Zubereitung bewirken kann. I m Zusammenhang mit dem Vis kositätsverlust ändern sich üblicherweise auch die sensorischen Eigenschaften der Zubereitung. Die kosmetischen Zubereitungen fühlen sich beispielsweise sensorisch weniger gehaltvoll (wässrig, leer) bzw. unvorteilhaft dünn an oder vermitteln einen geringeren Pflegecharakter. Allerdings unterliegen die Veränderungen der Eigenschaften des Produktes erheblichen Schwankungen in Abhängigkeit von z. B. der Stärke und der Schnelligkeit der Betätigung des Spenders durch den Anwender sowie der Temperatur und der Ausgangsviskosität der Zubereitung. Dies führt dazu, daß die Produkteigenschaften durch den Hersteller nur in begrenzter Weise so beeinflußt werden können, daß der Anwender bei der Anwendung tatsächlich die gewünschte bzw. eine reproduzierbare, über die gesamte Anwendungsdauer und für möglichst alle Anwender gleichbleibende Produktqualität erhält.

Aufgabe der vorliegenden Erfindung war es daher, die Nachteile des Standes der Technik zu vermeiden und insbesondere kosmetische oder dermatologische Zubereitungen zu finden, welche vor und nach der Applikation mit Hilfe von Schleppkolbenspendersystemen möglichst dieselben kosmetischen Eigenschaften aufweisen. Insbesondere sollten Zubereitungen gefunden werden, welche als Grundlage für Zubereitungsformen mit vielfältigen Anwendungszwecken dienen können und sich darüber hinaus durch sehr gute sensorische Eigenschaften, wie beispielsweise die Verteilbarkeit auf der Haut oder das Einzugsvermögen in die Haut auszeichnen.

Es war überraschend und für den Fachmann nicht vorauszusehen, dass die Verwendung von mindestens 0,01 Gew.-% eines oder mehrerer Hydrokolloide in kosmetischen oder dermatologischen Zubereitungen zur Verhinderung eines Strukturverlusts der Zubereitungen bei Entnahme aus Spendersystemen, in welchen die Zubereitungen bei der Entnahme einer Scherung ausgesetzt werden den Nachteilen des Standes der Technik abhelfen würden.

Bevorzugte Spendersystemen im Sinne der vorliegenden Erfindung sind Schleppkolbenspender, beispielsweise die in der WO-03/004374-A1 beschriebenen Schleppkolbenspender.

In einer besonders bevorzugten Ausführungsform der vorliegenden Erfindung ist das Spendersystem dahingehend ausgebildet, daß die Fördereinrichtung ein zu dem Behälter und dem Kopfstück längsverschiebliches Förderelement umfaßt, welches einen in der Förderkammer gleitverschieblichen Förderkolben aufweist, der mit einem Förderschaft verbunden ist, welcher einen Förderkanal umfänglich umgibt, der eine mit der Förderkammer kommunizierende Förderkanaleinlaßöffnung und eine Förderkanalauslaßöffnung aufweist, die durch eine Verschiebebewegung des Förderelementes relativ zu dem Kopfstück in eine Stellung bringbar ist, in der sich die Förderkanalauslaßöffnung zu dem Ausgabekanal öffnet.

Bei dem bevorzugten Spender öffnet sich die Förderkammer zu dem Ausgabekanal über eine Förderkanalauslaßöffnung, die über eine Längsverschiebung des Förderelementes relativ zu dem Kopfstück freigegeben wird. Diese Relativbewegung wird vorzugsweise dadurch erzielt, daß das Kopfstück handbetätigt wird, d. h. in axialer Richtung in Richtung auf den Behälter gleitverschoben wird. Der Durchlaß des pastösen Produktes von der Förderkammer zu der Produktabgabeöffnung am Ende des Ausgabekanals wird dementsprechend bereits durch eine translatorische Bewegung des Kopfstückes relativ zu dem Förderelement freigegeben. Ein vorheriger Druckaufbau in der Förderkammer, wie er beim gattungsbildenden Stand der Technik zur Freigabe des Durchganges erforderlich war, ist nicht erforderlich. Dementsprechend werden die Betätigungskräfte zur Abgabe von pastösen Produkten aus dem Spender verringert.

Bei dem bevorzugten Spender ist nachgeordnet zu der Förderkammer ein Förderkanal vorgesehen, der von einem Förderschaft umgeben ist. Am Ende dieses Förderkanals wird das aus der Förderkammer ausgeförderte pastöse Produkt durch die Förderkanalauslaßöffnung in den Ausgabekanal abgegeben. Erst nach Abgabe des Produktes aus der Förderkanalauslaßöffnung steht dieses in dem Ausgabekanal an.

Der verbleibende Ausgabekanal ist jedenfalls kürzer als bei den üblicherweise verwendeten Spendern. Dementsprechend wird deutlich weniger Volumen pastöser Masse durch eventuelle Oxidationsvorgänge beeinträchtigt. Die verbleibende Restlänge des Ausgabekanals kann insbesondere bei solchen Produkten, die gegenüber Oxidation hochgradig anfällig sind, dadurch verkürzt werden, daß der Ausgabekanal sich in Verlängerung der Stirnseite des Kopfstückes nach außen öffnet.

Bei einer vorteilhaften Ausgestaltung des bevorzugten Spenders wird die Förderkanalauslaßöffnung an der Umfangsfläche des Förderschaftes ausgespart und an dem Kopfstück eine die Förderkanalauslaßöffnung in der Ausgangsstellung der Fördereinrichtung abdeckende Buchse vorgesehen, so daß sich bei einer Hubbewegung des Kopfteiles zum Ausfördern pastöser Masse auf einfache Weise eine Freigabe der Förderkanalauslaßöffnung dadurch ergibt, daß der Förderschaft relativ zu der Buchse bewegt wird. Diese bevorzugte Ausgestaltung ist nicht nur einfach, sondern erlaubt auch eine Anordnung der Förderkanalauslaßöffnung in unmittelbarer Nähe der Einlaßöffnung des Ausgabekanals für das zu fördernde Produkt.

Mit Rücksicht auf eine gute axiale Führung der Fördereinrichtung relativ zu dem Kopfstück wird die vorerwähnte Buchse vorzugsweise als Führungsbuchse für die Fördereinrichtung ausgebildet und hat wenigstens eine mit der Umfangsfläche des Förderschaftes zusammenwirkende Führungsfläche.

Im Hinblick auf einen zwangsläufigen Verschluß der Förderkanalauslaßöffnung bei Rückstellung des Kopfstückes in die Ausgangslage wird gemäß einer bevorzugten Weiterbildung der vorliegenden Erfindung vorgeschlagen, daß an dem Kopfteil und an der Fördereinrichtung Mitnehmermittel vorgesehen sind, durch welche die Fördereinrichtung nach Handbetätigung bei Rückstellung des Kopfteiles in die Ausgangsstellung mitgenommen wird.

Die vorerwähnten Mitnehmermittel sind auf einfache Weise vorzugsweise durch eine an der Buchse ausgebildete Mitnehmerschulter gebildet, die mit einem an dem Förderschaft angeformten Mitnehmerkranz zusammenwirkt. Dieser Mitnehmerkranz ist vorzugsweise endseitig an dem Förderschaft angeformt, so daß die unterhalb des Mitnehmerkranzes ausgesparte Förderkanalauslaßöffnung in der Ausgangsstellung durch Anlage des Mitnehmerkranzes an Wandungen des Kopfteiles abgedichtet werden kann.

Bei der vorerwähnten bevorzugten Ausgestaltung kann das in dem Ausgabekanal anstehende Volumen dadurch weiter verringert werden, daß die Mitnehmerschulter endseitig an der Buchse und am Übergang zu dem Ausgabekanal und der Mitnehmerkranz im stirnseitigen Endbereich des endseitig verschlossenen Förderschaftes ausgebildet ist, wie dies gemäß einer bevorzugten Weiterbildung der vorliegenden Erfindung vorgeschlagen wird. Bei dieser bevorzugten Ausgestaltung deckt die endseitig an dem Förderschaft angeordnete Schaftkappe den Ausgabekanal in der Ausgangsstellung der Fördereinrichtung im wesentlichen bündig ab und weist vorzugsweise den Mitnehmerkranz auf.

Gemäß einer bevorzugten Weiterbildung der vorliegenden Erfindung erfolgt die Betätigung des Förderkolbens vorzugsweise über die endseitigen Stirnflächen der Führungsbuchse. Bei dieser bevorzugten Weiterbildung überragt der Förderkolben den Förderschaft radial zur Ausbildung einer ringförmigen Anlagefläche für eine Druckfläche, die stirnseitig an der Führungsbuchse ausgebildet ist und die in der Ausgangsstellung mit axialem Abstand zu der Anlagefläche angeordnet und durch axiales Verschieben des Kopfstückes in Richtung auf den Behälter an die Anlagefläche anlegbar ist.

Ebenfalls mit Rücksicht auf eine konstruktive Vereinfachung wird gemäß einer weiteren bevorzugten Ausgestaltung der vorliegenden Erfindung vorgeschlagen, die Innenwandung der Förderkammer durch eine Innenhülse auszubilden, welche an der kopfstückseitigen Stirnseite des Behälters vorgesehen ist. Dabei überragt die Innenhülse die Stirnseite des Behälters an der dem Kopfstück zugewandten Seite. Vorzugsweise ist die Innenhülse zur Verringerung der Bauteile einstückig an dem Behälter angeformt.

Zur einfachen Zentrierung des Kopfstückes bei der Montage des Spenders und leichten Befestigung des Kopfstückes an dem Behälter wird gemäß einer bevorzugten Weiterbildung der vorliegenden Erfindung ein Kopfgegenstück vorgeschlagen, das einen topfförmig auf die vorerwähnte Innenhülse gestülpten Haltezylinder sowie einen konzentrisch zu dem Haltezylinder angeordneten, die Gleitverschiebung des Kopfstückes führenden Führungszylinder aufweist. Der Führungszylinder und/oder der Haltezylinder erlauben eine leichte konzentrische Ausrichtung des Kopfteiles zu dem Zylinder. Ferner verbessert der Führungszylinder die Führung der Hubbewegung des Kopfstückes bei der Betätigung des Spenders.

Bei einer weiteren bevorzugten Ausgestaltung des bevorzugten Spenders, bei der das förderkammerseitige Ende des Führungszylinders einen Förderkolbenanschlag für den Förderkolben ausbildet, wird zum einen eine relativ längliche Führung für den Förderkolben und zum anderen auf einfache Weise eine Hubbegrenzung des Förderkolbens bereitgestellt. Eine derartige Hubbegrenzung hält beispielsweise das Kopfteil in der Ausgangslage an dem Behälter fest, wenn die Mitnehmermittel in Wirkverbindung stehen.

Vorzugsweise weist der Haltezylinder eine bodenseitige Ringschulter auf, welche eine Anlagefläche für eine Schraubenfeder ausbildet, die das Kopfstück in der Ausgangsstellung unter Vorspannung hält. Dies bietet den Vorteil, daß die äußere Umfangsfläche des Haltezylinders die Schraubenfeder innenseitig umgibt und somit ein Knicken der Feder verhindert. Die Ringschulter ist bei dieser bevorzugten Ausgestaltung auf die Stirnseite des Behälters aufgesetzt und ist somit insbesondere geeignet, das Kopfgegenstück in axialer Richtung gegenüber dem Behälter festzulegen.

Gemäß einer weiteren, besonders bevorzugten Ausgestaltung sind das Kopfgegenstück und das Kopfstück als vorgefertigte Spenderkomponente ausgebildet. Hierbei sind besonders bevorzugt das Kopfstück und das Kopfgegenstück mit ihrer äußeren Mantelfläche jeweils topfförmig übereinander geschoben, wobei das Kopfgegenstück wenigstens einen Anschlag zur Begrenzung der axialen Verschiebebewegung des Kopfstücks relativ zu dem Kopfgegenstück aufweist. In dem von den Mantelflächen umgebenden Innenraum befindet sich bei einer derartigen Ausgestaltung vorzugsweise ein Rückstellelement, beispielsweise die vorstehend erwähnte Schraubenfeder, welche das Kopfstück und das Kopfgegenstück in axialer Richtung beabstandet unter Vorspannung hält. Der vorerwähnte Anschlag grenzt die axiale Verschiebebewegung des Kopfstücks, d. h. sorgt nach dem Zusammenbau von Kopfstück und Kopfgegenstück unter Einschluß der Feder für den Zusammenhalt der beiden gegeneinander verschieblichen Bauteile. Die derart gebildete Spenderkomponente kann auf unterschiedlich ausgestaltete Behälter aufgesetzt werden, was eine wirtschaftliche Herstellung des Spenders für sehr unterschiedliche Anwendungen und Behältervolumina erlaubt.

Eine besonders einfache und haltbare Verbindung zwischen der vorgefertigten Spenderkomponente und dem Behälter wird dadurch gebildet, daß die Spenderkomponente mit dem Behälter über an dem Kopfgegenstück und der Stirnseite des Behälters ausgebildete Rastmittel verrastet sind.

Vorzugsweise ist bei dem bevorzugten Spender das Kopfstück derart längenverschieblich, daß das Kopfstück mittels Handbetätigung von der Ausgangsstellung zunächst um eine erste axiale Wegstrecke zur Anlage an den Förderkolben bei gleichzeitiger Freilegung der Förderkanalauslaßöffnung in dem Ausgabekanal in eine Mittelposition bringbar ist und das Kopfteil danach bei fortschreitender axialer Verschiebung unter Mitnahme des Förderkolbens von der Mittelposition in eine Ausgabe-Endposition bringbar ist, in welcher die Förderkammer durch Verschiebung des Förderkolbens ihr kleinstes Volumen erreicht hat. Bei dieser bevorzugten Ausgestaltung erfolgt das Freilegen der Förderkanalauslaßöffnung und das Komprimieren der Substanz in dem Förderkanal im Rahmen einer gleichgerichteten Bewegung des Kopfstücks in Richtung auf den Behälter. Diese bevorzugte Ausgestaltung erlaubt eine konstruktiv einfache Lösung des bevorzugten Spenders, bei welcher das Kopfstück unmittelbar auf den Förderkolben wirkt und diesen nach Freilegung der Förderkanalauslaßöffnung zum Fördern pastöser Masse antreibt. Diese Bewegung des Kopfstücks erfolgt üblicherweise gegen die Kraft eines Vorspannelementes, beispielsweise einer Feder, die dafür Sorge trägt, daß bei einer Entlastung des Kopfstücks dieses von der Ausgabe-Endposition weg von dem Behälter drückt. Bei dieser Bewegung wird zuerst die axiale Wegstrecke a zurückgelegt, d. h. die Förderkanalauslaßöffnung wird wieder verschlossen. Bei dieser Verschließbewegung findet eine Relativbewegung zwischen dem Förderschaft und dem Ausgabekanal statt, bei welcher das Volumen des Ausgabekanals an dessen Einlaß vergrößert wird. Dadurch wird die in dem Ausgabekanal befindliche pastöse Masse in Richtung auf die Pumpkammer zurückgezogen, also von der Produktabgabeöffnung des Ausgabekanals in dem Kopfteil entfernt.

Gemäß einer besonders bevorzugten Ausgestaltung befindet sich an dieser Produktabgabeöffnung ein Verschließteil. Das Verschließteil ist vorzugsweise derart beschaffen, daß es sich aufgrund einer Druckdifferenz zwischen dem Ausgabekanal und der Atmosphäre zur Abgabe des pastösen Produktes öffnet. Wird - wie vorstehend erwähnt - die pastöse Masse in dem Ausgabekanal weg von der Produktabgabeöffnung zurückgezogen, so führt dies zu einem relativen Unterdruck in dem Ausgabekanal, welcher dafür sorgt, daß das Verschließteil die Produktabgabeöffnung besonders wirkungsvoll abdichtet.

Im Hinblick auf eine möglichst gute Abdichtung ist es zu bevorzugen, die Produktabgabeöffnung um einen in dem Ausgabekanal angeordneten Verschlußdorn auszubilden.

Dieser Verschlußdorn ist vorzugsweise einstückig an dem Kopfteil ausgeformt. Das ebenfalls ringförmig ausgebildete Verschlußteil weist eine an dem Verschlußdorn dichtend anlegbare Dichtlippe auf, welche bei einem wirksamen Unterdruck den Ausgabekanal wirkungsvoll verschließt, jedoch beim Ausfördern des pastösen Produktes eine verhältnismäßig große Produktabgabeöffnung freigibt, durch welche bei relativ geringem Druckverlust das Produkt ausgefördert werden kann.

Besonders wirtschaftlich läßt sich ein Verschließteil hoher Wirksamkeit mittels Zweikomponenten-Spritzgießen an dem Kopfteil ausformen, wie dies gemäß einer bevorzugten Weiterbildung der vorliegenden Erfindung vorgeschlagen wird. Bei dieser Ausgestaltung ist das Verschließteil fest mit dem Kopfteil verbunden. Vorzugsweise ist das Verschließteil aus einem weichelastischen Kunststoff, besonders bevorzugt aus einem thermoplastischen Elastomer gebildet. Es hat sich gezeigt, daß insbesondere durch einen thermoplastischen Elastomer eine wirkungsvolle Abdichtung der Produktabgabeöffnung erreicht werden kann.

Es hat sich gezeigt, daß das Material für das Dichtteil sich besonders bevorzugt zur Ausbildung einer Funktionsfläche an der stirnseitigen Außenfläche des Kopfteiles genutzt werden kann. Eine derartige Funktionsfläche kann beispielsweise eine die haptischen Eigenschaften verbessernde Drückerfläche sein, gegen welche der Benutzer des Spenders bei dessen Benutzung drückt. Eine derartige Funktionsfläche wird vorzugsweise durch einen Überzug zumindest stirnseitig an der Außenseite des Kopfteiles ausgebildet. Das Verschließteil sowie der Überzug sind einstückig ausgeformt, vorzugsweise mittels Zweikomponenten-Spritzgießen nach der spritzgießtechnischen Herstellung des Kopfteiles.

Weitere Einzelheiten, Vorteile und Merkmale der vorliegenden Erfindung ergeben sich aus der nachfolgenden Beschreibung eines Ausführungsbeispiels in Verbindung mit der Zeichnung. In dieser zeigen :
Figur 1 eine Längsschnittansicht eines ersten Ausführungsbeispieles eines bevorzugten Spenders ;
Figur 2 eine Längsschnittansicht eines zweiten Ausführungsbeispiels eines bevorzugten Spenders.

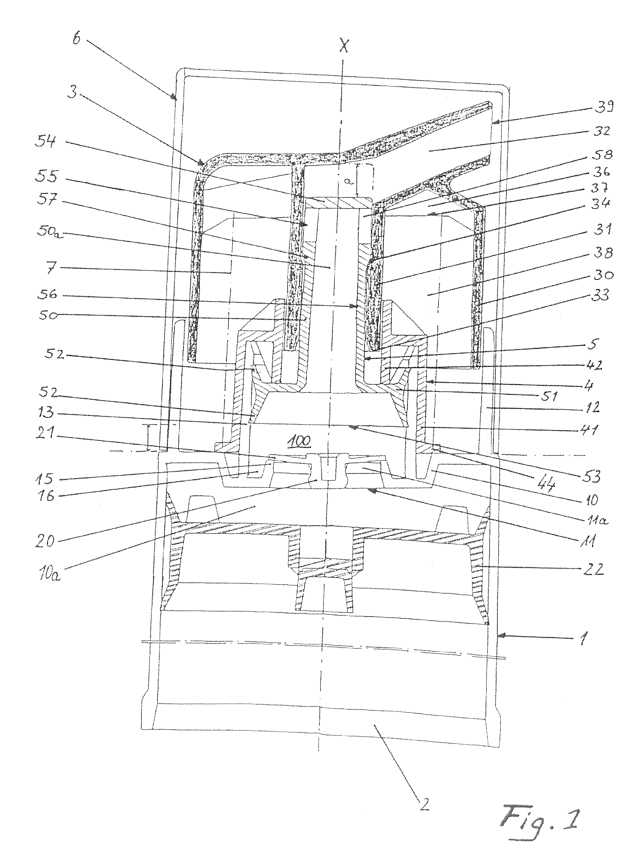

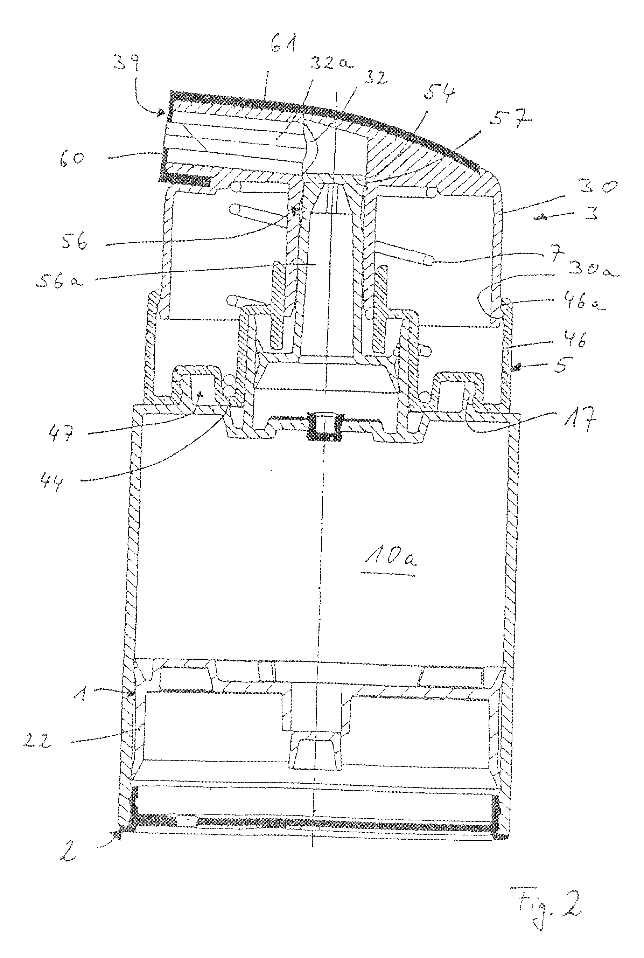

Das in Figur 1 gezeigte Ausführungsbeispiel eines bevorzugten Spenders hat einen Behälter 1, der topfförmig ausgebildet ist und an seiner Unterseite mit einer Bodenplatte 2 verbunden ist, welche mit dem Behälter 1 verrastet ist. An seiner anderen Stirnseite weist der Behälter 1 eine kopfseitige Abdeckung 10 auf, in der eine Behälteröffnung 11 ausgespart ist. Diese Abdeckung 10 ist auf der dem Behälter 1 abgewandten Seite zur Aufnahme eines Spenderkopfes bestehend aus einem Kopfstück 3, einem Kopfgegenstück 4 und einem Druckkolben 5 ausgebildet. Der Spender weist ferner eine auf eine sich oberhalb der Abdeckung 10 erstreckende Außenhülse 12 des Behälters 1 aufgeschobene Verschlußkappe 6 auf. Der Behälter 1, die Bodenplatte 2, das Kopfgegenstück 4 sowie der Druckkolben 5 sind als rotationssymmetrische Bauteile ausgebildet und zu einer Mittellängssachse X konzentrisch angeordnet. Zwischen dem Kopfstück 3 und dem Kopfgegenstück 4 befindet sich eine schematisch angedeutete Schraubenfeder 7, durch welche das Kopfstück 3 in der in Figur 1 gezeigten Ausgangsstellung gegenüber dem Kopfgegenstück 4 vorgespannt gehalten ist.

Das Kopfstück 3 weist einen zylindrischen Außenmantel 30 auf, welcher radial innerhalb unmittelbar benachbart zu der Außenhülse 12 des Behälters 1 und konzentrisch zu dieser angeordnet ist. Die Außenhülse 12 des Behälters 1 überragt das behälterseitige Ende des Außenmantels 30 in axialer Richtung. Dementsprechend erscheint das in Figur 1 gezeigte Ausführungsbeispiel des Spenders auch bei abgenommener Verschlußkappe als geschlossene Einheit bestehend aus dem Behälter 1 und dem Kopfstück 3. Wie nachfolgend näher erläutert wird, ist das Kopfstück 3 sowie der Druckkolben 5 längsverschieblich gegenüber dem Behälter 1 gehalten, wobei der Druckkolben 5 darüber hinaus längsverschieblich gegenüber dem Kopfstück 3 ist.

Die zylindrische Wandung des Behälters 1 umschließt einen Innenraum 10a zur Aufnahme der kosmetischen oder dermatologischen Zubereitung im Sinne der vorliegenden Erfindung. In der Behälteröffnung 11 erstrecken sich sternförmig ausgerichtete Haltestege 11 a. Auf der dem Innenraum 10a abgewandten Seite der Abdeckung 10 ist konzentrisch zu der Behälteröffnung 11 eine zylindrische Innenhülse 13 angeordnet, die in axialer Richtung von der Außenhülse 12 überragt wird und die eine Förderkammer 100 umgibt. Die Innenwandung der Innenhülse 13 ist glatt. Der Boden der Förderkammer 100 wird durch die Abdeckung 10 des Behälters 1 gebildet. Die Abdeckung 10 weist einen in die Förderkammer 100 hineinragenden Ringkranz 15 auf, welcher die Behälteröffnung 11 umgibt und zwischen sich und der Innenhülse 13 einen Ringspalt 16 bildet.

Der Druckkolben 5 weist einen im wesentlichen zylindrischen, innen hohlen Förderschaft 50 auf, an dessen einem Ende ein Förderkolben 51 einstückig angeformt ist. Der Förderkolben 51 überragt den Förderschaft 50 radial und hat an seiner äußeren Umfangsfläche jeweils obere und untere Dichtlippen 52, die den im wesentlichen ringförmig ausgebildeten Förderkolben 51 in axialer Richtung überragen. Auf einer dem Förderschaft 50 zugewandten Stirnseite bildet der Förderkolben 51 eine ringförmige Anlagefläche aus.

Der Förderschaft 50 weist an seinem einen Ende eine in der Mitte des ringförmigen Förderkolbens 51 ausgesparte Förderkanaleinlaßöffnung 53 auf. An seinem anderen Ende ist der Förderschaft 50 stirnseitig durch eine Schaftkappe 54 verschlossen. Die Schaftkappe 54 deckt einen Zylinderabschnitt 55 des Förderschaftes 50 ab, der gegenüber dem übrigen Schaftbereich 56 im Durchmesser vergrößert ist. Zwischen diesem Schaftbereich 56 und dem Zylinderabschnitt 55 befindet sich ein schräg nach außen geneigter Mitnehmerkranz 57. Zwischen dem Mitnehmerkranz 57 und der Schaftkappe 54 sind an der äußeren Umfangsfläche des Zylinderabschnitts 55 mehrere Förderkanalauslaßöffnungen 58 verteilt ausgespart. Zwischen den Förderkanalauslaßöffnungen 58 erstrecken sich in Umfangsrichtung Haltestege, welche die Schaftkappe 54 tragen. Die Förderkanaleinlaßöffnung 53 kommuniziert über einen von dem Förderschaft 50 umgebenden Förderkanal 50a mit den Förderkanalauslaßöffnungen 58 und bildet eine Förderpassage für die pastöse Substanz, die frei von Rückschlagventilen ist.

Das Kopfstück 3 weist einen, zylindrischen Außenmantel 30 auf, zu dem konzentrisch eine innen hohle Führungsbuchse 31 angeordnet ist, die mit einem Ausgabekanal 32 kommuniziert. Das stirnseitige Ende der Führungsbuchse 32 bildet eine stirnseitige Druckfläche 33 aus, welche in axialer Richtung von dem Außenmantel 30 überragt wird. Die Führungsbuchse 31 weist benachbart zu der stirnseitigen Druckfläche 33 einen ersten Buchsenabschnitt auf, der einen geringeren Innendurchmesser hat, als der in aus Förderrichtung der pastösen Substanz dahinterliegender zweiter Buchsenabschnitt. Zwischen dem ersten und dem zweiten Buchsenabschnitt ist eine Mitnehmerschulter 34 ausgebildet, welche die beiden Abschnitte unterschiedlicher Buchsendurchmesser über eine Schräge miteinander verbindet. Der zweite Buchsenabschnitt mündet in einen in den von der Mittellängsachse X seitlich abgehenden Ausgabekanal 32.

In etwa rechtwinkliger Erstreckung zu der Mittellängsachse X weist das Kopfstück 3 an Rippen 36 ausgebildete Federanlageflächen 37 auf. Die Rippen 36 erstrecken sich in etwa sternförmig von der Buchse 31 zu der Innenfläche des Außenmantels 30. Dementsprechend wird zwischen der Innenfläche des Außenmantels 30, der Außenfläche der Führungsbuchse 31 und dem Federanlageflächen 37 ein zu der Unterseite des Kopfstückes 3 offener Ringraum 38 ausgebildet.

Das Kopfteil 3 ist zu der behälterwärtigen Seite des Außenmantels 30 offen und oberhalb dieser Stirnseite im wesentlichen nach Art einer Kappe ausgebildet. An der der Stirnseite des Außenmantels 30 abgewandten Oberseite des Kopfteiles 3 befindet sich eine Produktabgabeöffnung 39 des Ausgabekanals 32.

Das Kopfgegenstück 4 weist im wesentlichen zwei konzentrische Zylinderabschnitte aufweist, nämlich einen äußeren Haltezylinder 41 und einen im Durchmesser kleineren Führungszylinder 42. Der Haltezylinder 41 überragt den Führungszylinder 42 auf der dem Behälter 1 zugewandten Seite, wohingegen der Führungszylinder 42 den Haltezylinder 41 auf der anderen Seite überragt. An der dem Behälter 1 abgewandten Stirnseite des Haltezylinders 41 ist ein radial von dort nach innen sich erstreckender Ringsteg vorgesehen, der etwa mittig an die Außenfläche des Führungszylinders 42 stößt.

Der Haltezylinder 41 weist an seiner behälterseitigen Stirnseite eine nach außen vorspringende umlaufende Ringschulter auf. Die behälterseitige Stirnseite des Führungszylinders 42 bildet einen Förderkolbenschlag aus.

Im zusammengebauten Zustand befindet sich der Förderkolben 51 des Druckkolbens 5 gleitverschieblich in der Innenhülse 13 des Behälters 1 und deckt somit stirnseitig die Förderkammer 100 ab. Das Kopfgegenstück 4 ist konzentrisch zu der Innenhülse 13 angeordnet und mit seinem Haltezylinder 41 topfförmig über die Innenhülse 13 geschoben. Die Ringschulter des Kopfgegenstücks 4 liegt an der dem Behälter 1 abgewandten Stirnseite der Abdeckung 10 an.

Die Ringschulter des Kopfgegenstückes 4 befindet sich etwa im Bereich des stirnseitigen Endes der Innenhülse 13. Der sich hieran radial nach innen anschließende Führungszylinder 42 umgibt Ende der Führungsbuchse 31 des Kopfteils 3. Radial innerhalb dieser Führungsbuchse 31 befindet sich der Förderschaft 50 mit seinem Schaftbereich 56 kleineren Durchmessers. Der Förderkolben 51 des Druckkolbens 5 ist gleitverschieblich an der Innenwandung der Innenhülse 13 angeordnet. Die ringförmige Anlagefläche des Förderkolbens 51 liegt stirnseitig an dem Förderkolbenanschlag des Führungszylinders 42 an. Hierdurch wird die von der Feder 7 auf das Kopfstück 3 ausgeübte Vorspannkraft gehalten, welche über die Anlage von Mitnehmerschulter 34 und Mitnehmerkranz 57 den Druckkolben 5 in einer Richtung weg von dem Behälter 1 vorspannt.

Zwischen der Förderkammer 100 und dem Innenraum 12 des Behälters 1 befindet sich ein in bekannter Weise ausgebildetes Behälterventil 20, das mit seiner Dichtungsscheibe 21 gegen den Ringkranz 15 der Abdeckung 10 anliegt und den Innenraum 10a gegenüber der Förderkammer 100 abdichtet.

Im nicht benutzen Zustand befindet sich der Spender in Ausgangsstellung (0).
Bei der Benutzung des Spenders drückt ein Benutzer das Kopfstück 3 in Richtung auf den Behälter 1. Aufgrund der Inkompressibilität des in der Förderkammer 100 und dem Förderkanal 50a enthaltenen Substanz verharrt der Druckkolben 5 in seiner Lage. Das Kopfstück 3 bewegt sich relativ zu dem Druckkolben 5 in Richtung auf den Behälter 1 zu. Die formschlüssige Anlage zwischen dem Mitnehmerkranz 57 und der Mitnehmerschulter 34 wird gelöst, bis die Schaftkappe 54 gegen die Innenfläche des Buchsenkopfes 35 stößt bzw. - je nach Ausgestaltung - die stirnseitige Druckfläche 33 am Ende der Führungsbuchse 31 an der ringförmigen Anlagefläche 51a des Förderkolbens 51 zur Anlage (Mittelposition M) kommt. Nach dieser axialen Verschiebung um den Verschiebeweg a liegen die Förderkanalauslaßöffnungen 53 in dem Ausgabekanal 32 frei.

Das Kopfstück 3 wird bei dieser wie auch bei jeder übrigen axialen Relativbewegung zwischen dem Kopfstück 3 und dem Kopfgegenstück 4 bzw. zwischen dem Kopfstück 3 und dem Behälter 1 durch die Anlage der Außenumfangsfläche der Führungsbuchse 31 an der Innenumfangsfläche des Führungszylinders 42 gleitend geführt. Die Relativbewegung zwischen dem Kopfstück 3 und dem Druckkolben 5 wird über die Anlage der Umfangsfläche des zweiten Schaftabschnittes an dem Schaftbereich 56 geführt.

Bei fortschreitender Druckbewegung des Kopfstückes 3 in Richtung auf den Behälter 1 wird der Druckkolben 5 mitgenommen. Hierbei verringert sich das Volumen der Förderkammer 100, so daß das sich in Förderrichtung hinter dem Behälterventil 30 befindende pastöse Produkt über die Förderkanalauslaßöffnung 53 in den Ausgabekanal 32 abgegeben wird. Das pastöse Produkt verläßt den Ausgabekanal über dessen Produktabgabeöffnung 39.

Am Ende dieser Relativbewegung des Kopfstückes 3 in Richtung auf den Behälter 1 stoßen die behälterseitigen Dichtlippen 52 des Druckkolbens 5 gegen die Stirnseite des Ringspalts 16. In dieser Ausgabe-Endposition V hat die Förderkammer 100 ihr kleinstes Volumen erreicht.

Wird nunmehr das Kopfstück 3 von dem Benutzer freigegeben, so drückt die Schraubenfeder 7 das Kopfstück 3 in entgegengesetzter Richtung zurück. Hierbei verharrt zunächst der Druckkolben 5 in seiner Ausgabe-Endposition V. Lediglich das Kopfstück 3 bewegt sich weg von dem Behälter 1, und zwar so lange, bis die Mitnehmerschulter 34 zur Anlage an den Mitnehmerkranz 57 kommt.

Bei dieser axialen Verschiebung um die Wegstrecke a wird das in dem Ausgabekanal 32 befindliche pastöse Produkt in den hierbei gebildeten Raum zwischen der Schaftkappe 54 und der Innenseite des Buchsenkopfes 35 zurückgezogen. Das pastöse Produkt liegt danach am Ende dieser Verschiebbewegung nicht mehr unmittelbar an der Produktausgabeöffnung 39 des Ausgabekanals 32 an, wodurch verhindert wird, daß pastöses Produkt am Ende des Fördervorgangs aus dem Ausgabekanal 32 tropft bzw. durch Verschmutzung im Bereich der Produktausgabeöffnung 39 beeinträchtigt wird.

Nach der Verlagerung um die Wegstrecke a und der Anlage von Mitnehmerkranz 57 und Mitnehmerschulter 34 wird auch der Druckkolben 5 bei fortschreitender Bewegung des Kopfstücks 3 in Richtung auf die Ausgangsstellung zurückbewegt, die dann erreicht wird, wenn der Förderkolbenanschlag gegen die ringförmige Anlagefläche des Förderkolbens 51 anliegt. Bei der Relativbewegung des Druckkolbens 5 weg von dem Behälter 1 wird pastöses Produkt aus dem Innenraum 10a des Behälters 1 durch die Behälteröffnung 11 in die Förderkammer 100 gefördert. Der hierbei in dem Innenraum 10a entstehende relative Unterdruck führt in an sich bekannter Weise zu einer Nachlaufbewegung des in dem Innenraum 12 befindlichen Nachlaufkolbens 22.

In Fig. 2 ist ein zweites Ausführungsbeispiel eines bevorzugten Spenders gezeigt.

Gleiche Teile sind bei diesem Ausführungsbeispiel gegenüber dem vorher Diskutierten mit gleichen Bezugszeichnen gekennzeichnet. Der Behälter 1 des in Fig. 7 gezeigten Ausführungsbeispiels ist im wesentlichen identisch wie der vorher beschriebene Behälter ausgebildet mit einer Behälteraußenwand, die einen Innenraum 10a umgibt, in dem ein Nachlaufkolben 22 längsverschieblich angeordnet ist und welcher von einer Bodenplatte 2 verschlossen ist. Im Gegensatz zu dem vorher beschriebenen Ausführungsbeispiel weist der Behälter 1 an seiner stirnseitigen Abdeckung einen umlaufenden Rastring 17 auf. Das Kopfgegenstück 4 ist über die Ringschulter 44 radial nach außen verlängert und hat eine sich im wesentlichen parallel zu dem Haltezylinder 51 erstreckende zylinderförmige Außenwand 46, deren Durchmesser größer als der Durchmesser des Außenmantels 30 des Kopfstücks 3 ist. Zwischen der Außenwand 46 und dem Haltezylinder 41 ist an der behälterwärtigen Unterseite des Kopfgegenstücks 4 eine Rastausnehmung 47 ausgeformt, welche mit dem Rastring 17 zur Ausbildung einer Rastverbindung zwischen dem Kopfstück 4 und dem Behälter 1 zusammenwirkt.

Bei dem in Fig. 2 gezeigten Ausführungsbeispiel ist das Kopfgegenstück 4 zusammen mit dem Kopfstück 3 als vorgefertigte Spenderkomponente ausgebildet. Das dem Behälter 1 abgewandte freie Ende der Außenwand 46 des Kopfgegenstücks 4 ist radial zur Ausbildung einer Rastnase 46a nach innen abgekröpft und überragt in axialer Richtung einen Ringwulst 30a, der an der Außenseite des Außenmantels 30 an dem Kopfstück 3 vorgesehen ist. Hierdurch ist ein Anschlag gebildet, durch welchen das Kopfgegenstück 4 unverlierbar mit dem Kopfstück 3 verbunden ist. Dieser Anschlag hält die von der Feder 7 aufgebrachten Federkräfte. Die das Kopfstück 3 und das Kopfgegenstück 4 umfassende Spenderkomponente kann somit vor der Montage auf den Behälter 1 vormontiert werden. Hierzu wird die Feder 7 in den Hohlraum zwischen dem Kopfstück 3 und dem Kopfgegenstück 4 eingesetzt. Die beiden Bauteile 3,4 werden so weit in axialer Richtung ineinander geschoben, bis der Ringwulst 30a an dem nach innen umbogenen Ende der Außenwand 46 vorbei geglitten ist.

Bei dem in Fig. 2 gezeigten Ausführungsbeispiel weist der Druckkolben 5 einen Mitnehmerkranz 57 auf, der an der Schaftkappe 54 ausgeformt ist. Dementsprechend dichtet der Mitnehmerkranz 57 bei der in Fig. 2 gezeigten Ausgangsstellung den Ausgabekanal 32 ab. Der Förderschaft 50 hat einen Schaftbereich 56 mit verkleinertem Durchmesser, dessen Längserstreckung der axialen Wegstrecke a entspricht. Dementsprechend wird die axiale Verschieblichkeit des Druckkolbens 5 gegenüber dem Kopfteil 3 durch die Schaftkappe 54 einerseits und die Längserstreckung des Schaftbereiches 56 mit verringertem Durchmesser andererseits festgelegt.

Bei dem in Fig. 2 gezeigten Ausführungsbeispiel besteht gegenüber dem vorerwähnten ersten Ausführungsbeispiel der weitere Unterschied, daß in dem Ausgabekanal 32 ein Verschlußdorn 32a vorgesehen ist, welcher einstückig an dem Kopfstück 3 ausgeformt ist. Durch den Verschlußdorn 32a wird die Produktabgabeöffnung 39 ringförmig. Die Produktabgabeöffnung 39 ist bei dem gezeigten Ausführungsbeispiel durch ein ringförmiges Verschließteil 60 abgedeckt, welches als separates Bauteil aus einem thermoplastischen Elastomer mit dem Kopfstück 3 verbunden ist. Das Verschließteil 60 liegt in der in Fig. 7 Ausgangsstellung an der äußeren Umfangsfläche und an Teilen der Stirnseite, insbesondere aber der Umfangsfläche des Verschlußdorns 32a an und dichtet somit den Ausgabekanal 32 ab. Einstückig mit dem Verschließteil 60 ist ein Überzug 61, der stoffidentisch wie das Verschließteil 60 ausgebildet ist und welcher sich über einen überwiegenden Teil der stirnseitigen Abdeckung des Kopfstücks 3 erstreckt. Durch diesen Überzug 61 wird eine rutschfeste Funktionsfläche an dem Kopfstück 3 ausgebildet.

Bei der Betätigung des in Fig. 2 gezeigten Spenders laufen die vorstehend insbesondere unter Bezugnahme auf die Fig. 1 erläuterten Vorgänge ab. Es besteht jedoch gegenüber dem vorerwähnten Ausführungsbeispiel vorliegend der Unterschied, daß beim Zurückstellen von Druckkolben 5 und Kopfstück 3 der Ausgabekanal gegenüber der Umgebung abgedichtet wird. Bei einer Relativbewegung des Druckkolbens 5 relativ zu dem Kopfstück 3 in Richtung auf den Behälter 1 zu, wird das in dem Ausgabekanal 32 befindliche Produkt - wie vorsteht bereits erwähnt - entgegen der Förderrichtung zurück in das Innere des Kopfstücks 3 gezogen. Bei dem in Fig. 2 dargestellten Ausführungsbeispiel bewirkt der hierbei entstehende Druckgradient zwischen der Atmosphäre und dem Ausgabekanal 32 ein vollständig dichtendes Anliegen des Verschließteiles 60 an den Flächen des Verschlußdornes 32a. Dementsprechend bleibt in dem Ausgabekanal 32 anliegendes pastöses Produkt nahezu unbeeinflußt von eventuellen Oxidationsvorgängen. Zusätzlich dichtet die Schaftkappe 54 den Förderkanal 50a gegenüber dem Ausgabekanal 32 ab, so daß insbesondere eine Beeinträchtigung in dem Förderkanal 50a befindlichen pastösen Produktes durch eventuell in den Ausgabekanal 32 eindringende Luft in jedem Fall vermieden wird.

Die beiden vorstehend beschriebenen Ausführungsbeispiele weisen gemeinsam den Vorteil auf, daß die Förderkanalöffnungen 58 erst nach einer Relativbewegung zwischen dem Kopfteil 3 und dem Druckkolben 5 in dem Ausgabekanal 32 freiliegen. Zum Fördern des pastösen Produktes aus der Förderkammer in Richtung auf die Produktabgabeöffnung 32a ist es nicht erforderlich, daß der zunächst aufgebaute Innendruck in der Förderkammer 100 dazu genutzt wird, ein in Förderrichtung dahinterliegendes Rückschlagventil zu öffnen. Dementsprechend kann das pastöse Produkt mit geringerem Kraftaufwand ausgefördert werden. Weiterhin bieten beide vorerwähnten Ausführungsbeispiele den Vorteil, daß das pastöse Produkt entgegen der Förderrichtung nach der Betätigung des Kopfstückes in dem Ausgabekanal 32 zurückgezogen wird, wobei das in Fig. 2 gezeigte Ausführungsbeispiel den zulässigen Vorteil hat, daß durch die dichtende Anlage des Verschließteiles 60 an dem Verschlußdorn 32a das in dem Spender enthaltene pastöse Produkt sicher vor einer Beeinträchtigung beispielsweise durch Sauerstoff in der Luft geschützt wird.

### Bezugszeichenliste:

| | |
|---|---|
| 1 Behälter | 34 Mitnehmerschulter |
| 2 Bodenplatte | 36 Rippe |
| 3 Kopfstück | 37 Federanlagefläche |
| 4 Kopfgegenstück | 38 Ringraum |
| 5 Druckkolben | 39 Produktabgabeöffnung |
| 6 Verschlußkappe | 41 Haltezylinder |
| 7 Schraubenfeder | 42 Führungszylinder |
| 10 Abdeckung | 44 Ringschulter |
| 10a Innenraum | 46 Außenwand |
| 11 Behälteröffnung | 46a Rastnase |
| 11a Haltesteg | 47 Rastausnehmung |
| 12 Außenhülse | 50 Förderschaft |
| 13 Innenhülse | 50a Förderkanal |
| 15 Ringkranz | 51 Förderkolben |
| 16 Ringspalt | 52 Dichtlippen |
| 17 Rastring | 53 Förderkanaleinlaßöffnung |
| 20 Behälterventil | 54 Schaftkappe |
| 21 Ventilscheibe | 55 Zylinderabschnitt |
| 22 Nachlaufkolben | 56 Schaftbereich |
| 30 Außenmantel | 57 Mitnehmerkranz |
| 30a Ringwulst | 58 Förderkanalauslaßöffnung |
| 31 Führungsbuchse | 60 Verschiießteil |
| 32 Ausgabekanal | 61 Überzug |
| 32a Verschlußdorn | 100 Förderkammer |
| 33 Druckfläche | |

Gegenstand der vorliegenden Erfindung sind daher ferner auch Kosmetik-Produkte, bestehend aus
a. einem Spender für pastöse Produkte mit einem das pastöse Produkt enthaltenden, im wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, dadurch gekennzeichnet daß die Fördereinrichtung ein zu dem Behälter (1) und dem Kopfstück (3) längsverschiebliches Förderelement (5) umfaßt, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlaßöffnung (53) und eine Förderkanalauslaßöffnung (58) aufweist, die durch eine Verschiebebewegung des Förderelementes (5) relativ zu dem Kopfstück (3) in eine Stellung bringbar ist, in der sich die Förderkanalauslaßöffnung (58) zu dem Ausgabekanal (32) öffnet, und
b. kosmetischen oder dermatologischen Zubereitungen, enthaltend mindestens 0,01 Gew.-% eines oder mehrerer Hydrokolloide.

Die Kosmetik-Produkte gemäß der vorliegenden Erfindung stellen in jeglicher Hinsicht überaus befriedigende Präparate dar, welche sich dadurch auszeichnen, daß die kosmetische oder dermatologische Zubereitung nach dem Austritt aus der Spenderöffnung dieselben kosmetischen Eigenschaften (wie z. B. Verteilbarkeit auf der Haut etc.) aufweist wie vor der Benutzung des Spenders bzw. daß ggf. leichte Veränderungen in den Eigenschaften der Zubereitung für den Anwender oder die Anwenderin nicht spür- bzw. fühlbar sind. Vorteilhaft ist die Viskositätsänderung der kosmetischen oder dermatologischen Zubereitung (vor und nach Entnahme aus dem Spender) weniger als 75 %, vorteilhaft weniger als 60 %, besonders vorteilhaft weniger als 50 %.
Die erfindungsgemäßen Produkte eignen sich ganz besonders, um als Grundlage für Produktformen mit vielfältigen Anwendungszwecken zu dienen.

"Hydrokolloid" ist die technologische Kurzbezeichnung für die an sich richtigere Bezeichnung "hydrophiles Kolloid". Hydrokolloide, auch Verdicker oder Gelbildner genannt, sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

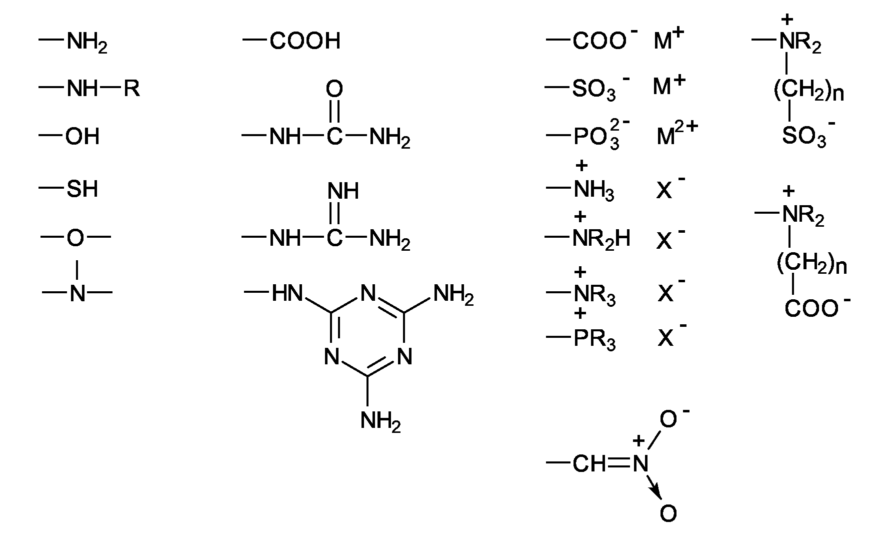

Die Gruppe der kosmetisch und dermatologisch relevanten Hydrokolloide läßt sich wie folgt einteilen in:
organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, organische, abgewandelte Naturstoffe, wie z. B. Carboxymethylcellulose und andere Celluloseether, Hydroxyethyl- und -propylcellulose und dergleichen, organische, vollsynthetische Verbindungen, wie z. B. Polyacryl- und Polymethacryl-Verbindungen, Vinylpolymere, Polycarbonsäuren, Polyether, Polyimine, Polyamide, anorganische Verbindungen, wie z. B. Polykieselsäuren, Tonmineralien wie Montmorillonite, Zeolithe, Kieselsäuren.

Als erfindungsgemäß vorteilhafte Hydrokolloide werden Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Celluloseether, Hydroxyethyl- und -propyl-cellulosederivate, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt.

Erfindungsgemäß bevorzugte Hydrokolloide sind beispielsweise auch alkylmodifizierte Cellulose-Derivate sowie Alkylhydroxycellulosen, wie z. B. Methylcellulosen, als welche die Methylether der Cellulose bezeichnet werden. Sie zeichnen sich durch die folgende Strukturformel aus 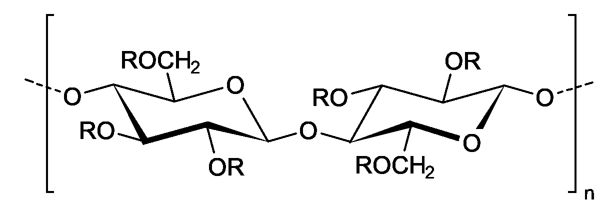 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Auch mikrokristalline Cellulose ist ein vorteilhaftes Hydrokolloid im Sinne der vorliegenden Erfindung. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11% aus Natrium Carboxymethyl Cellulose zusammensetzt. Weitere vorteilhafte Handelsprodukte dieser Rohstoffklasse sind Avicel® RC/ CL, Avicel® CE-15, Avicel® 500.

Bevorzugt im Sinne der vorliegenden Erfindung ist ferner Xanthan (CAS-Nr. 11138-66-2), auch Xanthan Gummi genannt. Vorteilhaftes Hydrokolloid im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet.

Vorteilhafte Hydrokolloide im Sinne der vorliegenden Erfindung sind ferner derivatisierte Gummen wie z.B. Hydroxypropyl Guar (Jaguar® HP 8) Hydroxpropyl Guar.

Auch Chitosan ist vorteilhaftes Hydrokolloid im Sinne der vorliegenden Erfindung. Chitosan ist gekennzeichnet durch folgende Strukturformel: 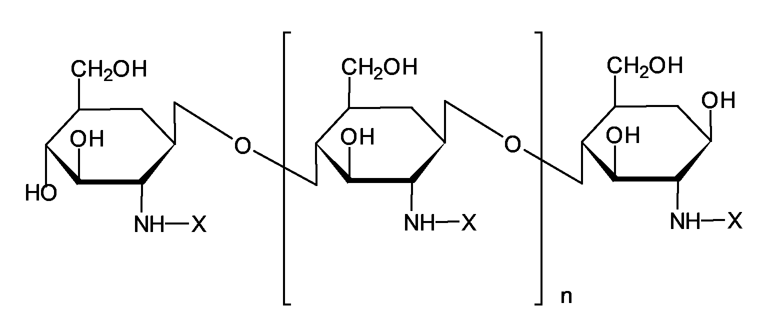 wobei n Werte bis zu ca. 10.000 annimmt und X entweder den Acetylrest oder Wasserstoff darstellt.

Erfindungsgemäß bevorzugt sind Chitosane mit einem Deacetylierungsgrad > 25 % , insbesondere > 55 bis 99 % [bestimmt mittels ¹H-NMR]).

Es ist von Vorteil, Chitosane mit Molekulargewichten zwischen 10.000 und 1.000.000 zu wählen, insbesondere solches mit Molekulargewichten zwischen 100.000 und 1.000.000. [bestimmt mittels Gelpermetionschromatographie].

Vorteilhafte Hydrokolloide im Sinne der vorliegenden Erfindung sind ferner Stärken, beispielsweise aus Mais, Weizen, Kartoffeln, Tapioca und Reis. Stärke besteht aus Amylose (zu ca. 20 bis 30 %) und Amylopektin (zu ca. 70 bis 80 %). Da insbesondere Amylose als Hydrokolloid im Sinne der vorliegenden Erfindung geeignet ist, ist es vorteilhaft, Pflanzenextrakte zu verwenden, die einen erhöhten Amyloseanteil besitzen.
Vorteilhafte Hydrokolloide im Sinne der vorliegenden Erfindung sind ferner modifizierte Stärken bzw. Stärkederivate. Hierzu zählen vernetzte, oxidierte, acetylierte, hydroxypropylierte und teilweise hydrolysierte Stärkemoleküle.

Besonders vorteilhaft sind ferner vorgelatinisierte, quervernetzte Stärkederivate wie Hydroxypropylstärkephosphat sowie hydroxypropylierte Phosphatester. Sie sind in der Regel "vorverkleistert" und liegen weitgehend in Form von "agglomerierten" Stärkekörnern vor. Aufgrund dieser Vorbehandlung ist Hydroxypropylstärkephosphat und deren Ester in kaltem Wasser vollständig löslich, nicht-ionisch, bevorzugt einsetzbar im alkalischen pH-Wertbereich, aber auch in einem breiteren pH-Wertbereich verwendbar. Erfindungsgemäß vorteilhafte Hydroxypropylstärkephosphate und Ester sind erhältlich unter der Handelsbezeichnung Structure ZEA, Structure XL von National Starch

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

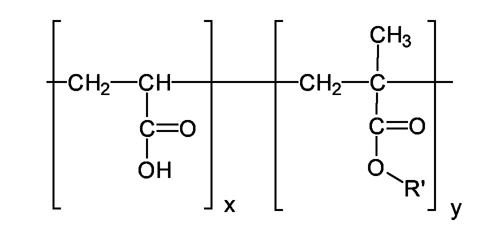

Darin stellen R' einen langkettigen Alkylrest und x und y Zahlen dar, welche den jeweiligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester.

Vorteilhaft sind Verbindungen, die die INCl-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 und Carbopol 1328, Ultrez 10, Ultrez 21 bei der NOVEON Inc sowie die unter der Handelsbezeichnung Tegocarbomer TC 341 ER von Goldschmidt / Degussa erhältlichen.

Weiterhin vorteilhafte Hydrokolloide im Sinne der vorliegenden Erfindung sind sog. AMPS-Polymere bzw -Copolymere, die durch radikalische Polymerisation bzw. Copolymerisation von Acrylamidomethylpropylsulfonsäure und optional einem oder mehreren olefinisch ungesättigten Comonomeren, die Sauerstoff, Stickstoff, Schwefel, Phosphor, Silizium, Chlor und/oder Fluor enthalten, sowie optional mit weiteren Makromonomeren, enthaltend eine zur Polymerisation befähigte Endgruppe und einen optionalen hydrophilen Teil, der z. B. auf Polyalkylenoxiden basiert, und einen optionalen hydrophoben Teil, der Wasserstoff oder einen gesättigten oder ungesättigten, linearen oder verzweigten, aliphatischen, cycloaliphatischen oder aromatischen (C₁-C₃₀)-Kohlenwasserstoffrest umfasst, herstellbar sind. Die Monomerenverteilung in den Polymeren kann hierbei beispielsweise alternierend, blockartig (auch Multiblock) oder auch statistisch (auch Gradient) sein. Die Polymere weisen im allgemeinen ein zahlenmittleres Molekulargewicht von 1000 bis 20.000.000 g/mol, bevorzugt 20.000 bis 5.000.000, insbesondere bevorzugt 100.000 bis 1.500.000 g/mol, auf.

In einer bevorzugten Ausführungsform sind die AMPS-Polymere vernetzt, d. h. sie enthalten mindestens einen Vernetzer mit mindestens zwei Doppelbindungen, der in das Polymer einpolymerisiert ist. Geeignete Vernetzer sind insbesondere Methylenbisacryl- und -methacrylamid, Ester ungesättigter Mono- oder Polycarbonsäuren mit Polyolen, z. B. Diacrylate oder Triacrylate, wie z. B. Butandiol- und Ethylenglykoldiacrylat bzw. -methacrylat und Trimethylolpropantriacrylat, Allylverbindungen, wie z. B. Allyl(meth)acrylat, Triallylcyanurat, Maleinsäurediallylester, Polyallylester, Tetraallyloxyethan, Triallylamin, Tetraallylethylendiamin, Allylester der Phosphorsäure und/oder Vinylphosphonsäurederivate.

Erfindungsgemäß vorteilhafte Verbindungen sind z.B. die Ammoniumacryloyldimethyltaurate/Vinylpyrrolidoncopolymere (Summenformel [C₇H₁₆N₂SO₄]ₙ [C₆H₉NO]ₘ) folgender statistischen Struktur entsprechend:

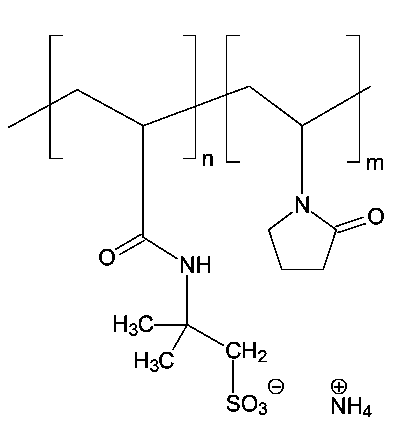

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.

Erfindungsgemäß vorteilhafte Hydrokolloide sind ferner sogenannte inverse Emulsionsverdicker, die in der Regel durch sog. Emulsionspolymerisation von gleichartigen oder verschiedenartigen, wasserlöslichen, hydrophilen Monomereinheiten, die in einem kosmetisch akzeptablen hydrophoben Medium dispergiert sind, unter optionaler Zugabe von Stabilisatoren (in der Regel Surfactants) erhalten werden. Beispiele hierfür sind z.B. Novemer EC1 von der Firma Noveon, die unter dem Handelsnamen Simulgel ® A, Simulgel ® EG, Simulgel ® EPG, Simulgel ® NS, Simulgel ® 600 und Sepigel ® 305 sowie Sepigel ® 501 von der Gesellschaft Seppic S.A. vertriebenen Rohstoffe, oder die Salcare SC91 und Salcare AST Typen der Firma Ciba.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCl-Bezeichnung "acrylates/steareth-20 methacrylate copolymer" (unter der Handelsbezeichnungen Aculyn® 22 bei der International Specialty Products Corp. erhältlich), die die INCl-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Die erfindungsgemäß besonders bevorzugten Hydrokolloide sind: Acrylates Copolymer (AQUA SF-1), Acrylates/C 10-30 Alkyl Acrylate Crosspolymer (Carbopol ETD 2020), Xanthan Gum (Kelter).

Das oder die anorganischen Hydrokolloide können beispielsweise vorteilhaft gewählt werden aus der Gruppe der modifizierten oder unmodifizierten, natürlich vorkommenden oder synthetischen Schichtsilikate.

Es ist zwar durchaus günstig, reine Komponenten einzusetzen, es können jedoch auch in vorteilhafter Weise, Gemische verschiedener modifizierter und/oder unmodifizierter Schichtsilikate eingesetzt werden.

Unter Schichtsilikaten, welche auch Phyllosilikate genannt werden, sind im Rahmen dieser Anmeldung Silikate und Alumosilikate zu verstehen, in welchen die Silikat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O- Valenz kann durch Kationen abgesättigt sein. Zwischen den einzelnen Schichten bestehen schwächere elektrostatische Wechselwirkungen, z. B. Wasserstoffbrückenbindungen. Das Schichtgefüge indessen ist weitgehend durch starke, kovalente Bindungen geprägt.

Die Stöchiometrie der Blattsilikate ist
(Si₂O₅²⁻) für reine Silicatstrukturen und
(AlₘSi²⁻ₘO₅(^{2+m})⁻) für Alumosilikate.
m ist eine Zahl größer als Null und kleiner als 2.

Liegen keine reinen Silikate sondern Alumosilikate vor, ist dem Umstande Rechnung zu tragen, daß jede durch Al³⁺ ersetzte Si⁴⁺ - Gruppe ein weiteres einfach geladenes Kation zur Ladungsneutralisierung erfordert.

Die Ladungsbilanz wird bevorzugt durch H⁺, Alkali- oder Erdalkalimetallionen ausgeglichen. Auch Aluminium als Gegenion ist bekannt und vorteilhaft. Im Gegensatz zu den Alumosilikaten werden diese Verbindungen Aluminiumsilikate genannt. Auch "Aluminiumalumosilikate", in welchen Aluminium sowohl im Silicatnetz, als auch als Gegenion vorliegt, sind bekannt und für die vorliegende Erfindung gegebenenfalls von Vorteil.

Schichtsilikate sind in der Literatur gut dokumentiert, z.B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H.Remy, 12. Aufl., Akademische Verlagsgesellschaft, Leipzig 1965, passim. Die Schichtenstruktur von Montmorillonit ist Römpps Chemie-Lexikon, Franckh'sche Verlagshandlung W. Keller & Co., Stuttgart, 8.Aufl., 1985, S. 2668 f., zu entnehmen.

### Beispiele für Schichtsilikate sind:

| | |
|---|---|
| Montmorillonit | Na_{0,33}((Al_{1,67}Mg_{0,33})(OH)₂(Sᵢ₄O₁₀)) |
| oft vereinfacht: | Al₂O₃*4SiO₂*H₂O*nH₂O bzw. Al₂[(OH)₂/Si₄O₁₀]· n H₂O |
| Kaolinit | Al₂(OH)₄(Si₂O₅) |
| llit | (K,H₃O)_{y}(Mg₃(OH)₂(Si_{4-y}A)_{y}O₁₀)) |
| und | (K,H₃O)_{y}(Al₂(OH)₂(Si_{4-y}Al_{y}O₁₀)) mit y = 0,7-0,9 |
| Beidellit | (Ca,Na)_{0,3}(Al₂(OH)₂(Al_{0,5}Si_{3,5}O₁₀)) |
| Nontronit | Na_{0,33}(Fe₂(OH)₂(Al_{0,33}S_{i3,67}O₁₀)) |
| Saponit | (Ca,Na)_{0,33}((Mg,Fe)₃(OH)₂(Al_{0,33}Si_{3,67}O₁₀)) |
| Hectorit | Na_{0,33}((Mg,Li)₃(OH,F)₂(Si₄O₁₀)) |

Montmorillonit stellt das Hauptmineral der natürlich vorkommenden Bentonite dar.

Sehr vorteilhafte anorganische Hydrokolloide im Sinne der vorliegenden Erfindung sind Aluminiumsilikate wie die Montmorillonite (Bentonite, Hectorite sowie deren Derivate wie Quaternium-18 Bentonit, Quaternium-18 Hectorite, Stearalkonium Bentonite bzw. Stearalkonium Hectorite) oder aber Magnesium-Aluminium-Silikate (Veegum®-Typen) sowie Natrium-Magnesium-Silikate (Laponite®-Typen).

Die oben angegebene chemische Formel ist nur angenähert, da Montmorillonite ein großes ionenaustausch-Vermögen besitzt, kann Al z. B. gegen Mg, Fe²⁺, Fe³⁺, Zn und andere ausgetauscht werden. Die daraus resultierende negative Ladung der Oktaeder-Schichten wird durch Kationen, insbesondere Na⁺ (Natrium-Montmorillonit) und Ca²⁺ in Zwischenschicht-Positionen ausgeglichen.

Im Sinne der vorliegenden Erfindung vorteilhafte synthetische Magnesiumsilikate bzw. Bentonite werden beispielsweise von Süd-Chemie unter der Handelsbezeichung Optigel® vertrieben.

Ein im Sinne der vorliegenden Erfindung vorteilhaftes Aluminiumsilikat wird beispielsweise von der R. T. Vanderbilt Comp., Inc., unter der Handelsbezeichnung Veegum® vertrieben. Die verschiedenen Veegum®-Typen, welche alle erfindungsgemäß vorteilhaft sind, zeichnen sich durch folgende Zusammensetzungen aus

| | (regular grade) | HV | K | HS | S-728 |
|---|---|---|---|---|---|
| SiO₂ | 55,5 | 56,9 | 64,7 | 69,0 | 65,3 |
| MgO | 13,0 | 13,0 | 5,4 | 2,9 | 3,3 |
| Al₂O₃ | 8,9 | 10,3 | 14,8 | 14,7 | 17,0 |
| Fe₂O₃ | 1,0 | 0,8 | 1,5 | 1,8 | 0,7 |
| CaO | 2,0 | 2,0 | 1,1 | 1,3 | 1,3 |
| Na₂O | 2,1 | 2,8 | 2,2 | 2,2 | 3,8 |
| K₂O | 1,3 | 1,3 | 1,9 | 0,4 | 0,2 |

Diese Produkte quellen in Wasser unter Bildung viskoser Gele, welche alkalisch reagieren. Durch Organophilierung von Montmorillonit bzw. Bentoniten (Austausch der Zwischenschicht-Kationen gegen quaternäre Alkylammonium-lonen) entstehen Produkte, welche als Bentone bezeichnet werden.

Bentone® ist eine Handelsbezeichnung für verschiedene neutrale und chemisch inerte Geliermittel, die aus langkettigen, organischen Ammoniumsalzen und speziellen Montmorillonit-Sorten aufgebaut sind. Bentone quellen in organischen Medien und bringen diese zum Quellen. Die Gele sind in verdünnten Säuren und Alkalien beständig, bei längerer Berührung mit starken Säuren und Alkalien verlieren sie ihre Geliereigenschaften jedoch teilweise. Aufgrund ihres organophilen Charakters sind die Bentone nur schwer durch Wasser benetzbar.

Folgende Bentone® -Typen werden beispielsweise von der Gesellschaft Kronos Titan vertrieben: Bentone® 27, ein organisch modifiziertes Montmorillonit, Bentone® 34 (Dimethyldioctylammoniumbentonit), das nach US 2,531,427 hergestellt wird und wegen seiner lipophilen Gruppen besser im lipophilen Medium als in Wasser quillt, Bentone® 38, ein organisch modifiziertes Montmorillonit, ein cremefarbenes bis weißes Pulver, Bentone® LT, ein gereinigtes Tonmineral, Bentone® Gel MIO, ein organisch modifiziertes Montmorillonit, das in Mineralöl (SUS-71) feinst suspendiert angeboten wird (10 % Bentonit, 86,7 % Mineralöl und 3,3 % Netzmittel), Bentone® Gel IPM, ein organisch modifiziertes Bentonit, das in Isopropylmyristat suspendiert ist (10 % Bentonit, 86,7 % Isopropylmyristat, 3,3 % Netzmittel), Bentone® Gel CAO, ein organisch modifiziertes Montmorillonit, das in Ricinusöl aufgenommen ist (10 % Bentonit, 86,7 % Ricinusöl und 3,3 % Netzmittel), Bentone® Gel Lantrol, ein organisch modifiziertes Montmorillonit, das in Pastenform zur Weiterverarbeitung, insbesondere zur Herstellung kosmetischer Mittel bestimmt ist; 10 % Bentonit, 64,9 Lantrol (Wollwachsöl), 22,0 Isopropylmyristat, 3,0 Netzmittel und 0,1 p-Hydroxybenzoesäurepropylester, Bentone® Gel Lan I, eine 10 %ige Bentone® 27-Paste in einer Mischung aus Wollwachs USP und Isopropylpalmitat, Bentone® Gel Lan II, eine Bentonit-Paste in reinem, flüssigem Wollwachs, Bentone® Gel NV, eine 15 %ige Bentone® 27-Paste in Dibutylphthalat, Bentone® Gel OMS, eine Bentonit-Paste in Shellsol T. Bentone® Gel OMS 25, eine Bentonit Paste in Isoparaffinischen Kohlenwasserstoffen (Idopar® H), Bentone® Gel IPP, eine Bentonit-Paste in Isopropylpalmitat.

Es ist vorteilhaft im Sinne der vorliegenden Erfindung, wenn der Gehalt der erfindungsgemäßen Hydrokolloide (eine oder mehrere Verbindungen) aus dem Bereich von 0,05 Gew.-% bis 5 Gew.-%, bevorzugt von 0,1 Gew.-% bis 3 Gew.-% gewählt wird, jeweils bezogen auf das Gesamtgewicht der kosmetischen oder dermatolgischen Zubereitung.

Es ist vorteilhaft im Sinne der vorliegenden Erfindung, Hydrokolloidmischungen aus mindestens zwei verschiedenen Hydrokolloiden einzusetzen. Erfindungsgemäß besonders vorteilhaft sind Mischungen aus:
- Xanthan Gummi und Schichtsilikaten;
- Xanthan Gummi und Polyacrylsäuren;
- Schichtsilikaten und Polyacrylsäuren;
- Cellulosederivaten und Polyacrylsäuren;
- Cellulosederivaten und Schichtsilikaten;
- Ammoniumdimethyltauramide /Vinylformamide Copolymer und Polyacrylat;
- Ammoniumdimethyltauramide Ninylformamide Copolymer und Polyacrylamid;
- Xanthan Gummi und Polyacrylsäuren und Cellulosederivaten;
- C10-30 Alkyl Acrylate Crosspolymere und Xanthan Gummi;
- Carbomer und Xanthan Gummi;
oder
- 2 verschiedenen Carbomeren.

Die Zubereitungen im Sinne der vorliegenden Erfindung können bevorzugt neben einer oder mehrerer Ölphasen zusätzlich eine oder mehrere Wasserphasen enthalten und beispielsweise in Form von O/W-, W/O/W- oder O/W/O-Emulsionen vorliegen. Solche Formulierungen können vorzugsweise auch Feststoff-Emulsionen (d. h. Emulsionen, welche durch Feststoffe stabilisiert sind, z. B. Pickering-Emulsionen), Gelemulsionen (Gelemulsionen oder Gelcremes sind sensorisch besonders leichte Produkte mit einem niedrigen Gehalt an Emulgatoren, Struktur- bzw. Gerüstbildnern (z. B. Fettalkoholen) und Lipiden), Hydrodispersionen oder auch Gele sowie schäumende Tensidzubereitungen sein.

### Pickering- / feststoffstabilisierte Emulsionen

Besonders vorteilhaft im Sinne der vorliegenden Erfindung sind ferner kosmetische oder dermatologische Zubereitungen, welche nur durch feinstverteilte Feststoffteilchen stabilisiert sind. Solche "emulgatorfreien" Emulsionen werden auch als Pickering-Emulsionen bezeichnet.

In Pickering-Emulsionen kommt es zu einer Anreicherung des festen Stoffes an der Phasengrenze Öl/Wasser in Form einer Schicht, wodurch ein Zusammenfließen der dispersen Phasen verhindert wird. Von wesentlicher Bedeutung sind dabei insbesondere die Oberflächeneigenschaften der Feststoffpartikel, welche sowohl hydrophile als auch lipophile Eigenschaften zeigen sollten.

Vorteilhaft können die stabilisierenden Feststoffteilchen auch oberflächlich wasserabweisend behandelt ("gecoatet") sein, wobei ein amphiphiler Charakter dieser Feststoffteilchen gebildet werden bzw. erhalten bleiben soll. Die Oberflächenbehandlung kann darin bestehen, daß die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffteilchen wird vorzugsweise kleiner als 100 µm, besonders vorteilhaft kleiner als 50 µm gewählt. Dabei ist es im wesentlichen unerheblich, in welcher Form (Plättchen, Stäbchen, Kügelchen etc.) bzw. Modifikation die verwendeten Feststoffteilchen vorliegen.

Vorzugsweise werden die mikrofeinen Feststoffteilchen aus der Gruppe der amphiphilen Metalloxidpigmente gewählt. Vorteilhaft sind insbesondere:
- Titandioxide (gecoatet und ungecoatet): z. B. Eusolex T-2000 von der Fa. Merck, Titandioxid MT-100 Z von der Fa. Tayca Corporation
- Zinkoxide z. B. Z-Cote und Z-Cote HP1 von der BASF AG, MZ -300, MZ -500 und MZ-505M von der Fa. Tayca Corporation
- Eisenoxide

Des weiteren ist es vorteilhaft, wenn die mikrofeinen Feststoffteilchen aus der folgenden Gruppe gewählt werden: Bornitride, Stärkederivate (Tapioca Starch, Sodium Corn Starch Octynylsuccinat etc.), Talkum, Latexpartikel.

Es ist erfindungsgemäß vorteilhaft, wenn die feststoffstabilisierten Emulsionen deutlich weniger als 0,5 Gew.-% eines oder mehrerer Emulgatoren enthalten bzw. sogar gänzlich emulgatorfrei sind.

Vorteilhaft im Sinne der vorliegenden Erfindung sind ferner z. B. Formulierungen, die ein Emulgatorsystem enthalten, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß ferner vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

Die kosmetischen oder dermatologischen Formulierungen im Sinne der vorliegenden Erfindung können wie üblich zusammengesetzt sein und dem kosmetischen oder dermatologischen Lichtschutz, ferner zur Behandlung, Pflege und Reinigung der Haut, der Lippen, sowie von Hautanhangsgebilden (Nägel und/oder Haare) und als Schminkprodukt in der dekorativen Kosmetik dienen.

Als Zubereitungen von flüssiger bzw. relativ fester Konsistenz können diese als kosmetische Reinigungslotionen bzw. Reinigungscremes Verwendung finden, welche beispielsweise zum Entfernen von Schminken und/oder Make-up oder als milde Wasch- und Duschcreme - ggf. auch für unreine Haut - verwendet werden können. Derartige Reinigungszubereitungen können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Produktes zur Pflege des Haars bzw. der Kopfhaut vorliegen, insbesondere eines Produktes zum Einlegen der Haare, das beim Fönen der Haare verwendet wird oder eines Frisier- und Behandlungsproduktes.

Entsprechend ihrem Aufbau können kosmetische oder topische dermatologische Zusammensetzungen im Sinne der vorliegenden Erfindung, beispielsweise verwendet werden als Hautschutzcreme, Reinigungsmilch, Tages- oder Nachtcreme usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern des Schäumens, Farbstoffe, Pigmente, die eine färbende Wirkung haben, weitere Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamat enthaltende Mischungen (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden das oder die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha -Liponsäure, Phytoen, D-Biotin, Coenzym Q10, alpha-Glucosylrutin, Carnitin, Carnosin, natürliche und/oder synthetische Isoflavonoide, Kreatin,Kreatinin, Ta urin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere α-Glucosylrutin), Coenzym Q10, Vitamin A und/oder Derivate, Vitamin C und/oder Derivate, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Couperosis), Schlaffheit und Ausbildung von Falten und Fältchen, lokale Hyper-, Hypo- und Fehlpigmentierungen (z. B. Alters flecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Es ist besonders vorteilhaft im Sinne der vorliegenden Erfindung, wenn die Wirkstoffe in verkapselter Form vorliegen, so daß sie pysikalisch getrennt von den Formulierungsbestandteilen (oder weiteren, nicht kompatiblen Wirkstoffen) vorliegen. In diesem Zusammenhang sind - je nach Art der eingesetzten Wirkstoffes - permanente Verkapselungen, d. h. Kapseln, aus denen die Wirkstoffe nicht in die kosmetische Zubereitung oder die Haut abgegeben werden (vorteilhaft z. B. für UV-Filtersubstanzen) oder nicht permanente Verkapselungen denkbar.

Vorteilhafte Verkapselungen bestehen z. B. Kunststoffen. Es ist ferner vorteilhaft, die weiteren Wirkstoffe in Kollagenmatrices und anderen üblichen Verkapselungsmaterialien zu verkapseln, z. B. als Celluloseverkapselungen, in Gelatine, Wachsmatrices oder liposomal verkapselt. Insbesondere Wachs matrices wie sie in der DE-OS 43 08 282 beschrieben werden, haben sich als günstig herausgestellt. Besonders vorteilhafte Verkapselungsformen im Sinne der vorliegenden Erfindung sind ferner Cyclodextrinkomplexe der weiteren Wirkstoffe.

Vorteilhaft sind auch z. B. Verkapselungen, die nach der Sol-Gel-Mikrotechnologie erhältlich sind. Hierbei werden die Wirkstoffe in einer inerten Silica Membran eingeschlossen, letztendlich also in Glasperlen verkapselt. Vorteilhaft im Sinne der vorliegenden Erfindung können verkapselte Wirkstoffe auch in Form von wäßrigen Dispersionen eingesetzt werden.

Verkapselte Wirkstoffe eignen sich insbesondere zur Herstellung von besonders hautverträglichen (Sensitiv-) Produkten. Darüber hinaus ist es selbstverständlich vorteilhaft, potentiell hautreizende Wirkstoffe in verkapselter Form einzusetzen.

Ferner ist es auch vorteilhaft im Sinne der vorliegenden Erfindung, als "microbeads" bezeichnete Wirkstoffkapseln einzusetzen. Vorteilhafte "microbeads" sind z. B. die im folgenden aufgelisteten:

| **Handelsname** | **Hersteller** | **Zusammensetzung (INCl)** |
|---|---|---|
| Unispheres UEL-611 | Induchem | Lactose + Cellulose + Hydroxypropyl Methylcellulose + Cl 77707 + Tocopherolacetate |
| Unipheres RP-572 | Induchem | Lactose + Cellulose + Hydroxypropyl Methylcellulose + Panthenyl Triacetate + Cl 7360 |
| Unipheres UT-513 | Induchem | Lactose + Cellulose + Hydroxypropyl Methylcellulose + Cl 77707 + Tocopherol |
| Macrobeads | Wiblosan | Cetearyl Alkohol + Acrylates Copolymer + Paraffinum Liquidum + Microcristalline Cellulose + Bisabolo l+ Tocopherol Acetate + Cl 74260 |

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte, Schaumstabilisatoren, Elektrolyte.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelier baren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethyl hexyllaurat, 2-Hexyidecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 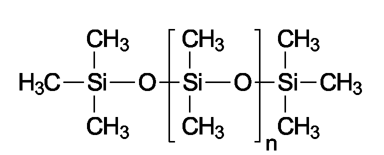 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCl: Phenyl Dimethicone, Phenyl Trimethicone), cyclische Silikone (Octamethylcyclotetrasiloxan bzw. Decamethylcyclopentasiloxan), welche nach INCl auch als Cyclomethicone bezeichnet werden, aminomodifizierte Silikone (INCl: Amodimethicone) und Silikonwachse, z. B. Polysiloxan-Polyalkylen-Copolymere (INCl: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäß der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, daß die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCl-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCl-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index,* 3. *Auflage, Society of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, -Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 -140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdikken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die Zubereitungen gemäß der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCl: Homosalate), 2-Ethylhexyl-2-cyano-3,3-diphenylacrylat (INCl: Octocrylene), 2-Ethylhexyl-2-hydroxybenzoat (2-Ethylhexylsalicylat, Octylsalicylat, INCl: Ethylhexyl Salicylate) und Ester der Zimtsäure, vorzugsweise 4-Methoxyzimtsäure(2-ethylhexyl)ester (2-Ethylhexyl-4-methoxycinnamat, INCl: Ethylhexyl Methoxycinnamate) und 4-Methoxyzimtsäureisopentylester (lsopentyl-4-methoxycinnamat, INCl: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |
| Eusolex T-aqua | Wasser/Alumina/Natriummetaphosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCl-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCl-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 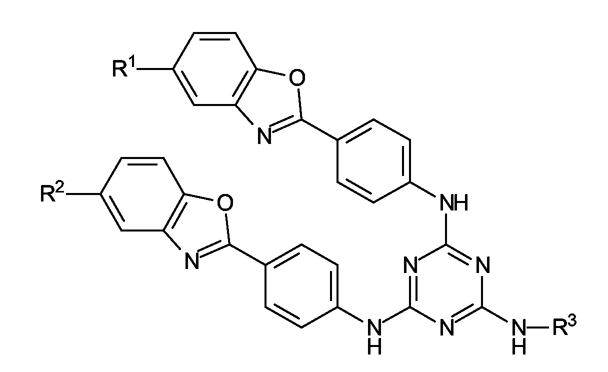 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 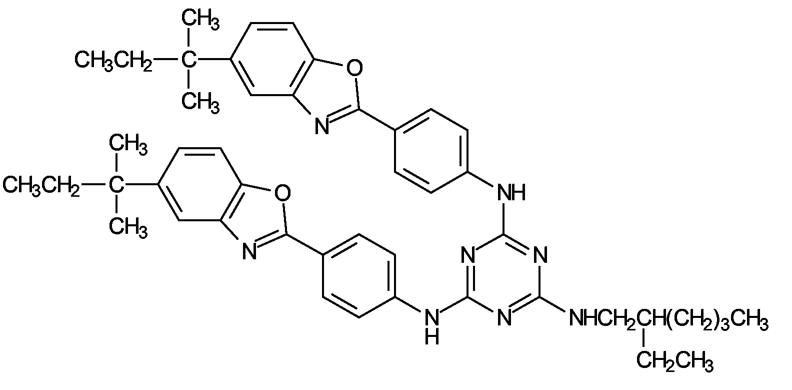 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 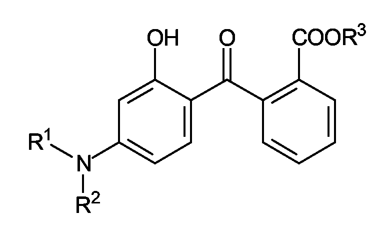 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 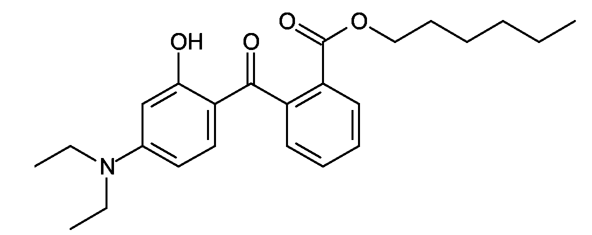 und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCl: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCl: Diethylhexyl Butamido Triazone), welches unter der Handelsbezeichnung UVASORB HEB bei Sigma 3V erhältlich ist;
- 4,4',4"-(1,3,5-Triazin-2,4,6-triyitriimino)-tris-benzoesäure-tris(2-ethylhexylester), auch: 2,4,6-Tris-[anilino-(p-carbo-2'-ethyl-1'-hexyloxy)]-1,3,5-triazin (INCl: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1 ,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCl: Methylene Bis-Benztriazolyl Tetramethylbutylphenol), welches z. B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCl-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.
Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die Zubereitungen im Sinne der vorliegenden Erfindung können ferner vorteilhaft Substanzen enthalten, die die Wasserfestigkeit der Produkte erhöhen, insbesondere wenn diese als Sonnenschutzprodukte verwendet werden sollen.

Vorteilhaft im Sinne der vorliegenden Erfindung sind PEG-45 Dodecylglykolcopolymer (INCl: PEG-45 Dodecyl Glycol Copolymer [y=z=11 und x=45]) und PEG-22 Dodecylglykolcopolymer (INCl: PEG-22 Dodecyl Glycol Copolymer [y=z=4,5 und x=22]) und Methoxy PEG-22 Dodecylglykolcopolymer (INCl: Methoxy PEG-22 Dodecyl Glycol Copolymer [y=7 und x=22 und R = CH₃]) zu verwenden, welche von der Firma AKZO Nobel erhältlich sind.

Vorteilhaft sind ferner beispielsweise wasserlösliche oder in Wasser dispergierbare Polyoxyethylen-Polyoxypropylen-Blockpolymere (CTFA-Bezeichnung: Polaxamere, CAS-Nr. 9003-11-6) mit folgender Struktur: 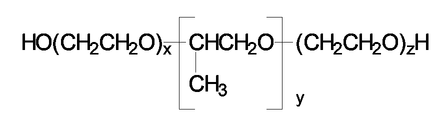 wobei x, y und z ganze Zahlen aus dem Bereich von 2 bis 130, insbesondere von 15 bis 100 darstellen und x und z gleich sind, aber unabhängig von y gewählt werden.

Unter diesen sind insbesondere Polaxamer 188 [mit x = 75, y = 30 und z = 75], welches unter der Handelsbezeichnung Lutrol F 68 (alt: Pluronic F 68) von der Firma BASF zu beziehen ist, das Polyxamer 185 [mit x = 19, y = 30 und z = 19] (Lubrajel WA von ISP), das Polyxamer 235 [mit x = 27, y = 39 und z = 27] (Pluronic F 85 von BASF) und/oder das Polyxamer 238 [mit x = 97, y = 39 und z = 97] (Pluronic F 88 von BASF) vorteilhaft zu verwenden.

Weitere vorteilhafte Substanzen, welche zur Steigerung der Wasserfestigkeit beitragen können, aber in die Ölphase der Zubereitungen gemäß der vorliegenden Erfindung eingearbeitet werden, sind bestimmte Wachskomponenten, wie acetyliertes Glykolstearat mit Tristearin (z. B. Unitwix von ISP mit der INCl: Acetylated Glycol Stearat and Tristearin), C₁₈₋₃₆ Fettsäuretriglycerid (z. B: Syncrowax HGLC von der Fa. Crode GmbH mit der INCI: C18-36 Acid Triglyceride) sowie die unter den Handelsbezeichnungen "Performa V 1608" (INCl: C30-38 Olefin/Isopropyl Maleate/MA Copolymer) und "Perfroma V 825" (synethisches Wachs) von New Phase Technologies erhältlichen Substanzen.

Es ist besonders vorteilhaft im Sinne der vorliegenden Erfindung die genannten Substanzen miteinander zu kombinieren, um die Wasserfestigkeit der Zubereitungen noch weiter zu verbessern.

Die Zubereitungen gemäß der vorliegenden Erfindung können ferner vorteilhaft auch Tenside enthalten, insbesondere wenn sie in Form von schäumende n Tensidzubereitungen vorliegen.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische lonen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2) X⁻ = beliebiges Anion, z.B. Cl⁻

RNH₂⁺CH₂CH₂COO⁻ (bei pH=7)

RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wäßrigem Medium keine lonen.

### A. Anionische Tenside

Vorteilhaft zu verwendende anionische Tenside sind
Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. Acyllactylate, Lauroyllactylat, Caproyllactylat
   6. Alaninate
Carbonsäuren und Derivate, wie
   1. Carbonsäuren, beispielsweise Laurinsäure, Aluminiumstearat, Magnesiumalkanolat und Zinkundecylenat,
   2. Ester-Carbonsäuren, beispielsweise Calciumstearoyllactylat, Laureth-6-Citrat und Natrium PEG-4-Lauramidcarboxylat,
   3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,
Sulfonsäuren und Salze, wie
   1. Acyl-isethionate, z.B. Natrium-/ Ammoniumcocoyl-isethionat,
   2. Alkylarylsulfonate,
   3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
sowie
Schwefelsäureester, wie
   1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
   2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkylamidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1.) O/W Emulsionen

| | **1** | **2** | **3** | **4** | **5** | **6** | **7** |
|---|---|---|---|---|---|---|---|
| PEG-40 Castor Oil, Natrium Cetearyl Sulfat Cetearyl Alkohol | | | | | | | 2,50 |
| Glycerinmonostearat (SE) | 1,00 | 2,00 | 3,00 | 1,00 | | 1,50 | |
| Glyceryl Stearat Citrat | | | | | 2,00 | | |
| Stearinsäure | 3,00 | | 2,50 | 2,00 | | | |
| PEG-40 Stearat | | 2,00 | | | | 2,00 | |
| PEG-100 Stearat | | | 0,75 | | | | |
| Lauryl Methicon Copolyol | | | | 0,75 | | 0,50 | |
| Sorbitan Stearat | | | 0,75 | | | | |
| Cetyl Phosphat | | | 0,75 | | | | |
| Stearyl Alkohol | | | 3,00 | | 2,00 | 2,00 | 0,50 |
| Cetyl Alkohol | 1,00 | 2,00 | | | 0,50 | | 2,00 |
| UVASorb® K2A | | | | 4,00 | | 5,00 | |
| Uvinul® A Plus | 2,50 | | | 0,25 | 1,00 | 0,50 | |
| Butylmethoxydibenzoylmethan | | | | | | | 4,00 |
| 4-Methylbenzylidencampher | | | 3,00 | | | | |
| Bis-Ethylhexyloxyphenol Methoxyphenyl Triazin | 1,00 | | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | | | 3,00 |
| Ethylhexyl Triazon | | | | 2,00 | | 2,00 | |
| Diethylhexyl Butamido Triazon | 2,00 | | | | | | |
| Ethylhexyl Methoxycinnamat | 3,50 | | | 10,00 | | | |
| Octocrylen | | | | 5,00 | 9,00 | 7,50 | 2,50 |
| Methylen Bis-Benztriazolyl | | | 2,00 | | 3,00 | | |
| Tetramethyl butyl phenol | | | | | | | |
| Ethylhexylsalicylat | | 0,50 | 3,00 | | | | 5,00 |
| Drometrizol Trisiloxan | | | 0,50 | | | 1,00 | |
| Terephtalidene Dicampher Sulfonsäure | | | | | | 2,00 | |
| Di methylcodiethyl benzal malonat | | | | | | 3,00 | |
| Titandioxid T 805 | 2,00 | | | | 1,00 | 0,50 | |
| Titandioxid MT-100Z | | | | 3,00 | 1,00 | | |
| Zinkoxid Z-Cote HP1 | | | | | | | |
| C₁₂₋₁₅ Alkyl Benzoat | 2,50 | | | | | 7,00 | 5,00 |
| Dicaprylylether | | | 3,50 | | 2,00 | | |
| Caprylsäure/Caprinsäuretriglycerid | | | | | | | |
| Paraffinöl | | 6,00 | | | | | |
| Butylenglycol Dicaprylat/ Dicaprat | | | | 5,00 | 3,00 | | |
| Cetearyl Isononanoat | 4,00 | | | | | 2,00 | 2,00 |
| Dimethicon | 0,50 | 3,00 | 1,00 | | 2,00 | | |
| Cyclomethicon | | 3,00 | | 4,50 | | | 0,50 |
| Dimethicon / Vinyl Dimethicon Crosspolymer | 4,00 | | | | | | 0,50 |
| PVP Hexadecen Copolymer | | | | 0,50 | 1,00 | | 1,00 |
| Glycerin | 7,50 | 10,00 | | 7,50 | 5,00 | | 20,00 |
| Xanthangummi | | 0,20 | 0,05 | | | | 0,30 |
| Polyacrylat (Carbomer) | 0,1 | | | 0,25 | 0,1 | 0,2 | |
| Butylenglycol | 5,00 | | | | | 7,00 | |
| Vitamin E Acetat | | 0,40 | 0,25 | 0,50 | 0,75 | | 1,00 |
| Dioic Acid | | | | 0,20 | | 0,25 | |
| Fucogel® 1000 | | | 1,50 | | | 5,00 | |
| Dihydroxyaceton | | 5,00 | | | | | |
| DMDM Hydantoin | 0,60 | | 0,40 | 0,20 | | | |
| Methylparaben | | 0,10 | 0,25 | | 0,50 | | |
| Phenoxyethanol | 0,40 | 0,50 | | 0,40 | 0,50 | | 0,60 |
| EDTA | 0,20 | | 0,35 | 0,50 | 0,02 | | 0,03 |
| Ethanol | 2,00 | | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 6,0-7,5 | 4,5-7,0 | 6,5-8,5 | 5,0-7,0 | 6,0-8,0 | 4,0-6,0 | 5,0-7,5 |

| | **8** | **9** | **10** | **11** | **12** |
|---|---|---|---|---|---|
| Glycerinmonostearat (SE) | | | | 1,00 | |
| Glyceryl Stearat Citrat | 2,00 | 2,00 | 1,50 | | |
| Polyglyceryl-3- Methylglucose Distearat | | | | | 4,50 |
| Stearyl Alkohol | 2,00 | 2,00 | | | |
| Cetyl Alkohol | | | 2,00 | | 4,50 |
| UVASorb® K2A | | | | | |
| Uvinul® A Plus | | | | | |
| Bis-Ethylhexyloxyphenol Methoxyphenyl Triazin | 2,00 | 2,00 | 1,50 | | |
| Dinatrium Phenyl Dibenzimidazol Tetrasulfonat | | | | | |
| Ethylhexyl Triazon | | | | | |
| Diethylhexyl Butamido Triazon | | 1,00 | 2,00 | | |
| Ethylhexyl Methoxycinnamat | 2,00 | 6,00 | 5,00 | | |
| Octocrylen | 2,00 | 9,00 | | | |
| Methylen Bis-Benztriazolyl Tetramethylbutylphenol | | | | | |
| Ethylhexylsalicylat | | | | | |
| Drometrizol Trisiloxan | | | | | |
| Titandioxid T 805 | | 3,00 | 2,00 | | |
| C₁₂₋₁₅ Alkyl Benzoat | | | 3,00 | 1,00 | 1,00 |
| Hydriertes Kokosfettsäureglycerid | 1,00 | 1,00 | 3,00 | | |
| Dicaprylylether | 5,00 | 2,00 | 6,00 | | |
| Octyldodecanol | 6,00 | 5,00 | 4,00 | 3,00 | 4,00 |
| Butylenglycol Dicaprylat/ Dicaprat | 5,00 | 2,00 | | | |
| Caprylsäure/Caprinsäuretriglycerid | | | | 2,00 | 5,50 |
| Dimethicon | | | 2,00 | | |
| Cyclomethicon | 2,00 | 1,00 | | 3,00 | |
| Sorbitol | | | | 2,50 | |
| Acrylat/C10-30 Alkyl Acrylat Crosspolymer | 0,10 | 0,10 | 0,05 | | |
| PVP Hexadecen Copolymer | 0,50 | 0,50 | | | |
| Glycerin | 8,00 | 6,00 | 5,00 | 3,00 | 3,00 |
| Xanthangummi | 0,40 | 0,40 | 0,25 | 0,30 | 0,10 |
| Butylenglycol | | | 3,00 | 3,00 | |
| Vitamin E Acetat | 0,50 | | 0,30 | 0,40 | 0,40 |
| Dihydroxyaceton | | | | 5,00 | 4,00 |
| DMDM Hydantoin | 0,60 | 0,60 | 0,50 | | |
| Methylparaben | | | | 0,30 | 0,30 |
| Phenoxyethanol | 0,40 | 0,40 | 0,35 | 0,50 | 0,50 |
| EDTA | 1,00 | 1,00 | 1,00 | | 1,00 |
| Ethanol | | | 3,00 | 3,00 | |
| Insekt Repellent 3535 | | | | | |
| Parfüm | 0,40 | 0,40 | 0,40 | 0,40 | 0,40 |
| Wasser | ad 100 | ad 100 | ad 100 | ad 100 | ad 100 |
| Neutralisationsmittel (Natrium- | qs | qs | qs | qs | qs |
| hydroxid, Kaliumhydroxid) | | | | | |
| pH-Wert | 5,0-7,0 | 5,0-7,0 | 5,0-7,0 | 5,0-7,0 | 5,0-7,0 |

| | **13** | **14** | **15** | **16** | **17** | **18** | **19** |
|---|---|---|---|---|---|---|---|
| Glyceryl Stearat, Ceteareth-12, Ceteareth-20, Cetearyl Alcohol, Cetyl Palmitat | | | | | | | 1,50 |
| Glycerinmonostearat (SE) | | 4,00 | | | | | |
| Glyceryl Stearat Citrat | 2,00 | | | | | | |
| Polyglyceryl-3- Methylglucose Distearat | | | | | 2,00 | | |
| Cetearyl Glucosid & Cetearyl Alkohol | | | | | | 2,00 | |
| Triceteareth-4 Phosphat | | | 1,20 | | | | |
| Trilaureth-4 Phosphat | | | | 2,00 | | | |
| Ceteareth-6 | | | 0,50 | 0,50 | | | |
| Sorbitan Stearat | | | 0,75 | 1,00 | 1,00 | | |
| Cetyl Phosphat | | | | | 2,00 | | |
| Stearyl Alkohol | | 2,50 | 3,00 | | | | 1,50 |
| Cetyl Alkohol | 2,50 | 1,00 | | | 0,50 | 2,00 | 2,00 |
| UVASorb® K2A | 1,00 | | | 4,00 | | 5,00 | |
| Uvinul®A Plus | 3,00 | 2,50 | 0,50 | 0,25 | 1,00 | 0,50 | |
| Butylmethoxydibenoylmethan | | | | | | | 4,50 |
| Bis-Ethylhexyloxyphenol Methoxyphenyl Triazin | | 2,00 | | | | 1,00 | 0,50 |
| Dinatrium Phenyl Dibenzimidazol Tetrasulfonat | | | | 1,00 | | 2,00 | |
| Phenylbenzmidazol Sulfonsäure | | | | | 3,00 | | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl Tetramethyl butyl phenol | | | 2,00 | | 3,00 | | |
| Ethylhexylsalicylat | | | | | | | 5,00 |
| Drometrizol Trisiloxan | | | 0,50 | | | 1,00 | |
| Terephtalidene Dicampher Sulfonsäure | | 3,00 | | | | | |
| Dimethylcodiethylbenzalmalonat | | | 5,00 | | | | |
| Titandioxid MT-100Z | 1,00 | | | 3,00 | 1,00 | | |
| Zinkoxid Z-Cote HP1 | | | | | | 3,00 | |
| Corapan TQ® | | | | | | | 6,00 |
| C₁₂₋₁₅ Alkyl Benzoat | | 2,50 | | | | 7,00 | 5,00 |
| Dicaprylylether | | | 3,50 | | 2,00 | | |
| Butylenglykol Dicaprylat/ Dicaprat | 5,00 | | | 5,00 | 3,00 | | |
| Cetearyl Isononanoat | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | 0,1 | 0,30 |
| Hydroxyethylcellulose | | 0,5 | | | 0,3 | 0,3 | |
| Carbomer | | | 0,05 | 0,2 | | | |
| Butylenglycol | 7,00 | 5,00 | | | | 7,00 | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Alkohol | | 2,00 | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (NaOH, KOH) | qs | qs | qs | qs | qs | qs | qs |
| pH-Wert | 4,5-6,0 | 4,5-7,0 | 5,5-7,5 | 5,0-7,0 | 5,5-7,5 | 4,0-7,0 | 4,0-7,5 |

### 2.) Schaumförmige O/W- Emulsionen:

| | **1** | | **2** | |
|---|---|---|---|---|
| | Gew.-% | Vol.-% | Gew.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglycerid | 4,00 | | 2,00 | |
| C12-15 Alkyl Benzoat | 10,00 | | 15,50 | |
| Cyclomethicon | 4,00 | | | |
| Dimethicon | | | 0,50 | |
| Octylisostearat | | | 5,00 | |
| Magnesium Aluminium Schichtsilikat | 0,2 | 0,1 | 0,05 | 0,5 |
| Carbomer | 0,2 | 0,1 | 0,2 | 0,1 |
| Myristyl Myristat | | | 2,00 | |
| Ceresin | 1,50 | | | |
| Glycerin | 5,00 | | 10,00 | |
| UVASorb® K2A | 2,00 | | | |
| Uvinul A Plus® | 2,00 | | 1,50 | |
| Terephthaliden Dicamphersulfonsäure | 0,50 | | | |
| Drometrizol Trisiloxan | 1,50 | | | |
| Ethylhexylmethoxycinnamat | 5,00 | | 4,00 | |
| Ethylhexyltriazon | | | 3,00 | |
| Octocrylene | 5,00 | | | |
| Titandioxid Uvinul T 805 | 1,00 | | | |
| BHT | | | 0,02 | |
| Na₂H₂EDTA | 0,50 | | 0,10 | |
| Parfüm, Konservierungsmittel, | q.s. | | q.s. | |
| Farbstoffe, usw. | q.s. | | q.s. | |
| Kaliumhydroxid | q.s. | | q.s. | |
| Wasser | ad 100,00 | | ad 100,00 | |
| | pH-Wert eingestellt auf 6,5-7,5 | | pH-Wert eingestellt auf 5,0-6,0 | |
| Emulsion 1 | | 70 | | |
| Emulsion 2 | | | | 35 |
| Gas (Stickstoff, Sauerstoff oder Kohlendioxid) | | 30 | | |
| Gas (Luft) | | | | 65 |

| **Emulsion** | **3** | **4** | **5** |
|---|---|---|---|
| Stearinsäure | 2,00 | 2,00 | |
| Palmitinsäure | | | 1,50 |
| Cetylalkohol | 2,50 | 2,00 | |
| Stearylalkohol | | | 3,00 |
| PEG-100 Stearat | | | 3,50 |
| PEG-40 Stearat | | 2,00 | |
| PEG-20-Stearat | 3,00 | | |
| Sorbitanstearat | | 0,80 | |
| C12-15 Alkyl Benzoat | 5,00 | | |
| C12-13 Alkyl Tartrat | | | 7,00 |
| Butylenglycol Dicaprylat/Dicaprat | | 6,00 | |
| Dicaprylyl Ether | | | 2,00 |
| Cyclomethicon | | 2,00 | 3,00 |
| Butylenglycol | 1,00 | | |
| Isohexadecan | 2,00 | | |
| Methylpropandiol | | | |
| Propylenglykol | | | 5,00 |
| Hydroxyethylcellulose | 0,1 | 0,05 | 0,1 |
| Magnesium Aluminium Schichtsilikate | 0,3 | 0,5 | 0,5 |
| Glycerin | 5,00 | 7,00 | |
| UVASorb® K2A | | | 2,00 |
| Uvinul A Plus® | 2,00 | | |
| NeoHeliopan® AP | | | |
| Phenylbenzimidazol Sulfonsäure | | | |
| Ethylhexylmethoxycinnamat | | | |
| Ethylhexyltriazon | 2,00 | 2,00 | 2,00 |
| Octocrylen | 2,00 | | |
| Bis-Ethylhexyloxyphenol Methoxyphenyltriazin | | 3,00 | 3,00 |
| Vitamin E Acetat | | 0,5 | |
| BHT | | | 0,10 |
| Na₂H₂EDTA | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

| **Emulsion** | **6** | **7** | **8** | **9** |
|---|---|---|---|---|
| Stearinsäure | 1,50 | | | |
| Palmitinsäure | | | 3,00 | 3,00 |
| Cetylalkohol | | 3,00 | | |
| Cetylstearylalkohol | | | 2,00 | 2,00 |
| Stearylalkohol | 3,00 | | | |
| PEG-100 Stearat | | 4,00 | | |
| PEG-40 Stearat | 3,00 | | | |
| PEG-20-Stearat | | | 3,00 | 3,00 |
| Sorbitanstearat | 1,00 | | | |
| Tridecyl Trimellitate | | 5,00 | | |
| C12-15 Alkyl Benzoat | | | 3,00 | 3,00 |
| Butylenglycol Dicaprylat/Dicaprat | 8,00 | | | |
| Octyldodecanol | | 2,00 | | |
| Kokosfettsäureglycerid | | | | 2,00 |
| Dicaprylyl Ether | | | 2,00 | 2,00 |
| Cyclomethicon | | | | |
| Dimethicon | 1,00 | | 2,00 | 2,00 |
| Isohexadecan | | 3,00 | | |
| Methylpropandiol | | 4,00 | | |
| Propylenglykol | | | | |
| Glycerin | 5,00 | | 6,00 | 6,00 |
| Carbomer | 0,1 | | 0,2 | |
| C10-30 Alkyl Acrylate Crosspolymer | | 0,25 | | 0,3 |
| NeoHeliopan® AP | | 2,00 | | |
| Phenylbenzimidazol Sulfonsäure | 1,00 | 4,00 | 1,00 | 1,00 |
| Ethylhexylmethoxycinnamat | 5,00 | | 4,00 | 4,00 |
| Ethylhexyltriazon | | | | |
| Diethylhexylbutamidotriazon | 1,00 | | | |
| Butyl Methoxydibenzoylmethan | 2,50 | | 2,00 | 2,00 |
| Bis-Ethylhexyloxyphenol Methoxyphenyltriazin | 2,00 | | | |
| Vitamin E Acetat | 0,20 | | 0,30 | 0,30 |
| BHT | | 0,05 | | |
| Na₂H₂EDTA | | | 0,40 | 0,40 |
| Parfüm, Konservierungsmittel | q.s. | q.s. | q.s. | q.s. |
| Farbstoffe, usw. | q.s. | q.s. | q.s. | q.s. |
| Natriumhydroxid | q.s. | q.s. | q.s. | q.s. |
| Kaliumhydroxid | | | | q.s. |
| Wasser | ad 100,0 | ad 100,0 | ad 100,0 | ad 100,0 |

Zur Herstellung des Schaums werden 80-97 Vol.-% der Emulsion I mit 3-20 Vol.-% eines geeigneten Gases (z.B. Propan/ Butan, Druckluft, Stickstoff) aufgeschäumt.

### 3.) Hydrodispersionen

| | **1** | **2** | **3** | **4** | **5** | **6** |
|---|---|---|---|---|---|---|
| | | | | | | |
| Glyceryl Stearat Citrat | | 0,40 | | | | |
| Cetyl Alkohol | | | | | 2,00 | |
| Natrium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | 0,30 | | 0,15 | | 0,50 |
| Dimethicon / Vinyl Dimethicon Crosspolymer | | | | 5,00 | | 3,00 |
| UVASorb® K2A | | | | | 3,50 | |
| Uvinul® A Plus | 0,25 | | | 0,50 | 2,00 | 1,50 |
| Butyl Methoxydibenzoylmethan | | | 3,50 | | | |
| Bis-Ethylhexyloxyphenol Methoxypheny Triazin | | 2,00 | | 0,25 | | |
| Terephthaliden Dicampher Sulfonsäure | | | | | | 0,50 |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Methylen Bis-Benzotriazolyl Tetramethylbutylphenol | | | | | | |
| Butyl Methoxydibenzoylmethan | | 3,50 | | | | |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| Titandioxid MT-100 Z | 0,50 | 2,00 | 1,00 | 2,00 | 3,00 | 1,00 |
| C₁₂₋₁₅ Alkyl Benzoat | 2,00 | | 2,50 | | | |
| C18-36 Triglycerid Fettsäure | | | 1,00 | | | |
| Butylenglycol DicaprylaUDicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α - Glycosylrutin | | | | | 0,25 | |
| Trinatrium EDTA | | 1,00 | 1,00 | 0,10 | 0,20 | |
| Dekaben LMB ® | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |
| pH-Wert | 4,5-5,5 | 5,0-7,0 | 5,0-7,0 | 5,0-7,0 | 4,0-6,0 | 5,0-7,5 |

### 4.) Gelcremes

| | **1** | **2** | **3** | **4** | **5** |
|---|---|---|---|---|---|
| Carbomer | 0,125 | 0,125 | 0,125 | 0,125 | 0,125 |
| Shea Butter | | | | 1,00 | |
| Mineralöl | | | | | 6,00 |
| Octyldodecanol | | | | 1,50 | |
| Caprylsäure/Caprinsäuretriglycerid | | | 4,00 | | |
| Dicaprylyl Carbonat | | 9,00 | | 1,00 | 3,00 |
| Dimethicon | | | | 0,50 | |
| Cyclomethicon | 9,00 | 2,00 | 3,00 | 2,00 | 1,00 |
| Diazolidinylharnstoff | 0,20 | 0,20 | 0,20 | 0,20 | 0,20 |
| Phenoxyethanol + Ethyl-, Methyl-, Propyl- Butyl-, Isobutylparaben | 0,50 | 0,50 | 0,50 | 0,50 | 0,50 |
| Parfum | | | | 0,25 | 0,25 |
| Glycerylstearatcitrat | 1,00 | 1,00 | 1,00 | 1,00 | 1,00 |
| Hydrierte Kokosfettsäureglycerid | 1,00 | 1,00 | 1,00 | 1,00 | 1,00 |
| Ammonium Acryloyldimethyltaurat/VP Copolymer | | | | | 0,125 |
| Hydroxyethylcellulose | 0,375 | 0,375 | 0,375 | 0,375 | 0,375 |
| Menthol | 0,10 | 0,50 | 1,00 | 0,10 | 0,10 |
| Wasser + Alcohol Denat | | | | 3,00 | |
| Wasser + Blue 1 | | 0,200 | 0,60 | 0,40 | |
| Wasser + Glycerin | 10,00 | 10,00 | 10,00 | 10,00 | 10,00 |
| Xanthangummi | 0,125 | 0,125 | 0,125 | 0,125 | |
| Wasser | ad 100,0 | ad 100,0 | ad 100,0 | ad 100,0 | ad 100,0 |
| Neutralisationsmittel (Natriumhydroxid, Kaliumhydroxid) | qs | qs | qs | qs | qs |
| pH-Wert | 4,5-5,5 | 6,5-8,5 | 5,0-7,0 | 4,0-6,0 | 5,0-7,5 |

### 5.) Feststoffstablisisierte Emulsionen

| | **1** | **2** | **3** | **4** | **5** |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 9,00 | 6,00 | | |
| C12-15- Alkyl Benzoat | | | | 5,00 | 8,00 |
| Butylene Glykol Dicaprylat/Dicaprat | | | | | 8,00 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,00 | 2,00 | 1,50 |
| Disteardimonium Hectorit | 1,00 | 0,750 | 0,50 | 0,50 | 0,25 |
| Cera Microcristallina + Paraffinöl | | | 2,50 | | 5,00 |
| Hydroxypropyl Methylcellulose | 0,15 | | | | 0,05 |
| Xanthan Gummi | | 0,3 | | | |
| Dimethicon | | | 4,50 | | |
| UVASorb® K2A | 2,00 | 5,00 | 3,00 | 1,50 | 1,00 |
| Bis-Ethylhexyloxyphenol Methoxypheny Triazin | 1,00 | 3,00 | 0,75 | 1,00 | 1,00 |
| Terephthaliden Dicampher Sulfonsäure | | 2,00 | | | 0,50 |
| Phenylbenzmidazol Sulfonsäure | 2,00 | 0,50 | 1,00 | | |
| Uvinul® A Plus | | | 2,75 | | 0,50 |
| Ethylhexylmethoxycinnamat | 6,00 | | | | 3,0 |
| Octocrylen | 3,50 | | 7,50 | | |
| Ethylhexyl Salicylat | | 3,50 | | | 4,00 |
| Diethylhexyl Butamido Triazon | | | | | 4,0 |
| Titandioxid Eusolex ® T-2000 | | 2,00 | 4,00 | 2,00 | 4,00 |
| Titandioxid T 805 ® | | | | | 3,00 |
| Silica Dimethyl Silylat | | | 1,00 | | |
| Bornitrid | 2,00 | | | 3,00 | |
| Tapioca Stärke | | | | 1,00 | |
| Natrium Chlorid | 1,00 | 1,00 | 1,00 | 1,00 | |
| Glycerin | 5,0 | 10,0 | | 6,00 | 10,0 |
| Trinatrium EDTA | | 1,00 | | 1,00 | |
| Methylparaben | 0,21 | | | | 0,20 |
| Propylparaben | 0,07 | | | | |
| Phenoxyethanol | 0,50 | | 0,40 | 0,40 | 0,50 |
| Hexamidin Diisethionat | | | | | 0,08 |
| Diazolidinyl Harnstoff | | | 0,28 | 0,28 | |
| Alkohol | | 5,00 | | 2,50 | |
| Parfüm | 0,45 | 0,20 | | | 0,45 |
| Wasser | ad 100 | ad 100 | ad 100 | ad 100 | ad 100 |

### 6.) Gele:

| **Eye Shadow Gel** | |
|---|---|
| | Gew.-% |
| PEG-8 (Polyethylenglycol 400) | 2,00 |
| Ethanol | 5,00 |
| Aristoflex AVC | 1,50 |
| Glycerin | 2,00 |
| Panthenol | 0,50 |
| Tocopherolacetat | 0,50 |
| Timiron Splendid blue ® (Merck KgaA) | 4,50 |
| Chromoxid grün | 1,00 |
| Parfüm, Konservierungsmittel, NaOH, Komplexbildner, Farbstoffe, Antioxidantien etc. | q.s. |
| Wasser | ad 100,00 |

| **Highlighter Gel** | |
|---|---|
| | Gew. % |
| Carbomer | 1,50 |
| Glycerin | 2,50 |
| 1,3 Butylenglycol | 2,50 |
| Glitzerpigmente (z.B. Helicone HC Scarabeus, Wacker) | 1,00 |
| EDTA | 0,20 |
| Dimethicone | 1,50 |
| Parfüm, Konservierungsmittel, NaOH, Farbstoffe, Antioxidantien, etc. | q.s. |
| Wasser | ad 100,00 |

| **Eye Liner Gel** | |
|---|---|
| | Gew. % |
| Perlglanzpigmente | 10,00 |
| Eisenoxid | 3,00 |
| Silica | 2,00 |
| Aristoflex AVC | 1,00 |
| Hyroxyproylethyl Cellulose | 0,35 |
| Citric Acid | q.s. |
| Glycerin | 5,00 |
| PVP / VA Copolymer | 2,00 |
| Parfüm, Konservierungsmittel, Farbstoffe, NaOH, Komplexbildner, Antioxidantien, etc. | q.s. |
| Wasser | ad 100,00 |

### 7.) Make-up

| **Emulsions-Make-up** | |
|---|---|
| | Gew. % |
| PEG-30 Stearat | 2,00 |
| Glycerinmonostearat | 1,00 |
| Stearinsäure | 1,00 |
| Cyclomethicon | 7,00 |
| Octyldodecanol | 7,00 |
| Isopropyl Lanolat | 4,00 |
| Squalan | 2,00 |
| Octyl Methoxycinnamat | 2,00 |
| Butyl Methoxydibenzoylmethan | 1,00 |
| Xanthan Gum | 0,20 |
| Glycerin | 5,00 |
| Butylenglkcol | 2,00 |
| Vitamin E Acetat | 1,00 |
| Magnesiumsilikat | 1,00 |
| Glimmer | 1,00 |
| Eisenoxid | 1,00 |
| Titandioxid | 2,50 |
| Talkum | 5,00 |
| EDTA | 0,50 |
| Parfüm, Konservierungsmittel, NaOH, Antioxidantien etc. | q.s. |
| Wasser | ad 100,00 |

| **Emulsions-Make-up** | |
|---|---|
| | Gew. % |
| Cyclomethicon and PEG /PPG -18/18 Dimethicon | |
| (z.B. Dow Corning 3225 Formulation Aid) | 10,00 |
| Cyclomethicon | 10,00 |
| Bienenwachs | 3,00 |
| Polyglyceryl-4 Oleat | 2,00 |
| Quetrnium-18 Hektorit | 0,50 |
| Mikrokrsitalline Cellulose | 0,50 |
| Eisenoxid | 1,00 |
| Titandioxid | 2,50 |
| Talkum | 12,00 |
| Natrium Chlorid | 2,00 |
| Parfüm, Konservierungsmittel, NaOH, Antioxidantien etc. | q.s. |
| Wasser | ad 100,00 |

| **Cover Cream** | |
|---|---|
| | Gew. % |
| Cyclomethicon | 44,00 |
| Bienenwachs | 3,00 |
| Carnaubawachs | 10,00 |
| Lanolinöl | 5,00 |
| Paraffinöl | 8,40 |
| Quaternium-18 Hectorit | 2,00 |
| Cetyl Alkohol | 2,60 |
| Eisenoxid | 3,00 |
| Titandioxid | 7,50 |
| Nylon | 6,00 |
| Talkum | 10,50 |
| Parfüm, Konservierungsmittel, Antioxidantien etc. | q.s. |

| **Emulsions-Make-up** | |
|---|---|
| | Gew. % |
| Cyclomethicon | 18,00 |
| Phenyltrimethicon | 3,00 |
| Cetyl PEG/PPG - 10/1 Dimethicon (z.B. Abil EM 90) | 4,00 |
| Paraffinöl | 3,00 |
| Mikrokrsitalline Cellulose | 0,50 |
| Eisenoxid | 2,30 |
| Titandioxid | 4,50 |
| Talkum | 2,00 |
| Natrium Chlorid | 2,00 |
| Quaternium-18 Hectorit | 0,30 |
| Propylen Carbonat | 0,08 |
| Parfüm, Konservierungsmittel, NaOH, Antioxidantien etc. | q.s. |
| Wasser | ad 100,00 |

### 8.) Reinigungsemulsion (O/W)

| | **1** | **2** | **3** | **4** | **5** |
|---|---|---|---|---|---|
| Natriumlaurylethersulfat | 10 | 9 | 8 | - | 10 |
| Natriummyrystylethsulfat | | | | 8 | |
| Alkylamidopropylbetain | - | 3 | 5 | 2 | - |
| Natriumacylglutamat | - | - | 2 | - | - |
| Alkylpolyglucosid | - | - | - | 2 | 1,5 |
| Acrylatcopolymer (Acrylates/C 10-30 Alkyl Acrylate Crosspolymer) | 0,65 | 0,5 | 0,3 | 0,6 | 0,7 |
| Phenoxethanol + Methylparaben + Butylparaben + Ethylparaben + Isobutylparaben + Propylparaben | 0,8 | 0,8 | 0,8 | 0,8 | 0,8 |
| Sojaöl | 6 | 40 | 25 | - | 30 |
| Paraffinöl | 35 | 5 | 18 | 45 | 13 |
| Mandelöl | 2 | - | - | - | - |
| Quaternäres Ammoniumsalz der Hydroxyethylcellulose | - | - | 0,10 | - | - |
| Ethoxylierte Glycerin-Fettsäureester (PEG-7 Glyceryl Cocoate) | - | 0,5 | - | 1 | 1 |
| Unispheres (Lactose + Cellulose + Hydroxypropyl Methylcellulose + Cl 77007) | 0,2 | - | - | - | - |
| NaOH | q.s. | q.s. | q.s. | q.s. | q.s. |
| Parfum | 1,0 | 1,2 | 1,0 | 1,0 | 0,8 |
| Wasser | ad 100 | ad 100 | ad 100 | ad 100 | ad 100 |

### 9.) Duschgel

| | **1** | **2** | **3** | **4** | **5** | **6** | **7** | **8** | **9** | **10** |
|---|---|---|---|---|---|---|---|---|---|---|
| Natriummyrystylethsulfat | - | - | - | - | - | - | - | - | - | 4 |
| Natriumlaurylethersulfat | 13,0 | 11,0 | 9,0 | 8,5 | 12,0 | 10 | 11 | - | 10 | - |
| Alkylamidopropylbetain | 0,50 | 1,5 | 2,0 | 1,0 | | 4,0 | 2,5 | 4,0 | 4,0 | 4,5 |
| Alkylpolyglucosid | - | - | - | - | 1,10 | - | - | 4,0 | | 1,0 |
| Natriumcocoylglutamat | 1,50 | 0,5 | 1,0 | 0,5 | 0,75 | - | 3,0 | 1,5 | 2,0 | - |
| Acrylatcopolymer (Acrylates Copolymer) | 3,00 | 1,50 | 1,75 | 2,00 | 2,20 | 2,40 | 3,5 | 2,8 | 2,4 | - |
| Quaternäres Ammoniumsalz der Hydroxyethylcellulose | _ | _ | - | 0,20 | - | - | - | - | - | |
| PEG-6 Caprylic/Capric Glyceride | - | 0,75 | 1,0 | - | - | 1,0 | - | - | | - |
| PEG-40 hydriertes Rizinusöl | 1,0 | 1,0 | 1,0 | 1,0 | 1,0 | 1,0 | 1,0 | 1,2 | 0,7 | - |
| PEG-200 hydriertes Glycerin Palmitinsäureester | - | - | - | 0,1 | - | - | - | - | - | 1,0 |
| Glycol Distearate | 1,0 | - | - | - | - | - | - | - | | - |
| Styrene/Acrylate Copolymer | - | 0,5 | - | - | - | - | - | - | 0,5 | - |
| DMDM Hydantoin | 0,30 | 0,30 | 0,30 | 0,30 | | 0,30 | 0,30 | 0,30 | | |
| Methylparaben | - | - | - | - | 0,40 | - | - | - | 0,40 | 0,40 |
| Propylparaben | - | - | - | - | 0,20 | - | - | - | 0,20 | 0,20 |
| Phenoxyethanol | - | - | - | - | 0,60 | - | - | - | 0,60 | 0,60 |
| Unispheres (Lactose + Cellulose + Hydroxypropyl Methylcellulose + Cl 77007) | 0,3 | - | - | - | - | 0,20 | - | - | - | 0,1 |
| Polyethylene | - | - | - | - | - | - | - | - | 0,2 | - |
| Wasser + Cl 42051 | - | 0,05 | - | - | - | - | - | - | - | - |
| Zitronensäure | q.s. | q.s. | q.s. | q.s. | q.s. | q.s. | q.s. | q.s. | q.s. | q.s. |
| NaOH | q.s. | q.s. | q.s. | q.s. | q.s. | q.s. | q.s. | q.s. | q.s. | q.s. |
| Parfum | 1,0 | 0,5 | 1,2 | 1,0 | 0,8 | 1,0 | 0,8 | 1,0 | 1,0 | 0,5 |
| Wasser | ad 100 | ad 100 | ad 100 | ad 100 | ad 100 | ad 100 | ad 100 | ad 100 | ad 100 | ad 100 |

### 10.) Reinigungsgel

| | **1** | **2** | **3** |
|---|---|---|---|
| Natriumlaurylethersulfat | 5 | | 3 |
| Natriummyrystylethsulfat | | 2 | |
| Alkylamidopropylbetain | - | 2 | 0,5 |
| Alkylpolyglucosid | 0,5 | - | 0,3 |
| Acrylatcopolymer (Carbomer) | 1,2 | 1,0 | 1,5 |
| Xanthangummi | 0,25 | 0,10 | - |
| Phenoxethanol + Methylparaben + Butylparaben + Ethylparaben + Isobutylparaben + Propylparaben | 0,8 | 0,8 | 0,8 |
| Glycerin | 1,5 | 1,0 | - |
| Mandelöl | - | 0,1 | - |
| Quaternäres Ammoniumsalz der Hydroxyethylcellulose | - | - | 0,10 |
| Ethoxylierte Glycerin-Fettsäureester (PEG-7 Glyceryl Cocoate) | 0,5 | 0,5 | - |
| Unispheres (Lactose + Cellulose + Hydroxypropyl Methylcellulose + Cl 77007) | - | - | 0,2 |
| NaOH | q.s. | q.s. | q.s. |
| Parfum | 0,5 | 0,6 | 1,0 |
| Wasser | ad 100 | ad 100 | ad 100 |

### 11.) Duschpeeling

| | **1** | **2** | **3** |
|---|---|---|---|
| Natriumlaurylethersulfat | 15 | 8 | 10 |
| Natriummyrystylethsulfat | - | 2 | - |
| Alkylamphoacetat | 4,0 | - | 5,0 |
| Alkylamidopropylbetain | - | 2 | 0,5 |
| Alkylpolyglucosid | - | - | 0,5 |
| Magnesium-Aluminium Silikate | 2,5 | 2,0 | 2,3 |
| Polyethylen | 5,0 | 2,5 | 5,0 |
| Phenoxethanol + Methylparaben + Butylparaben + Ethylparaben + Isobutylparaben + Propylparaben | 0,8 | 0,8 | 0,8 |
| Mandelöl | - | 0,1 | - |
| Quaternäres Ammoniumsalz der Hydroxyethylcellulose | 0,2 | - | 0,10 |
| Ethoxylierte Glycerin-Fettsäureester (PEG-7 Glyceryl Cocoate) | 0,5 | 0,5 | - |
| Unispheres (Lactose + Cellulose + Hydroxypropyl Methylcellulose + Cl 77007) | 0,2 | 0,2 | 0,2 |
| Zitronensäure | q.s. | q.s. | q.s. |
| Parfum | 0,8 | 0,6 | 1,0 |
| Wasser | ad 100 | ad 100 | ad 100 |

### 12.) Shampoos

| **Conditioner-Shampoo** | | | | | |
|---|---|---|---|---|---|
| | **1** | **2** | **3** | **4** | **5** |
| Natrium Laurylethersulfat | 9 | 9 | 9 | 9 | 9 |
| Cocamidopropyl Betain | 4 | 4 | 4 | 4 | 4 |
| Dinatrium PEG-5 Laurylcitrat Sulfosuccinat | 3 | 3 | 3 | 3 | 3 |
| Acrylatcopolymer (Acrylates Copolymer) | 2.0 | 2.0 | 3.0 | 3.0 | 3,0 |
| Polyquaternium-10 | 0.3 | 0.1 | 0.1 | 0.3 | - |
| Guar Hydroxypropyl-Trimonium Chlorid | - | - | 0.1 | 0.2 | 0.2 |
| Perlglanz | 1.5 | 3 | 4 | 2 | 2,5 |
| Trübungsmittel | - | - | - | - | 0.5 |
| Iminodibersteinsäure | 0.1 | 0.2 | 0.1 | 0.5 | 0.5 |
| PEG-40 hydriertes Rizinusöl | 0.2 | 0.2 | 0.2 | 0.2 | 0.2 |
| Unispheres (Lactose + Cellulose + Hydroxypropyl Methylcellulose + Cl 77007) | - | - | 0.3 | - | - |
| Natrium Salicylat | 0.4 | 0.4 | 0.4 | 0.4 | 0.4 |
| Natrium Benzoat | 0.4 | 0.4 | 0.4 | 0.4 | 0.4 |
| Natriumchlorid | 0,9 | 1,0 | 1,2 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

| **Klares Conditioning-Shampoo** | | | | | |
|---|---|---|---|---|---|
| | **1** | **2** | **3** | **4** | **5** |
| Natrium Laurylethersulfat | 10 | 9 | 3.5 | 3.5 | 0.5 |
| Natrium Myrethsulfat | - | - | 3.5 | 3.5 | 3.0 |
| Cocamidopropyl Betain | 4 | 4.5 | 3 | - | - |
| Natrium Cocoamphoacetat | - | - | - | 2.5 | - |
| Dinatrium PEG-5 Laurylcitrat Sulfosuccinat | - | - | - | - | 2.5 |
| Decylglucosid | - | - | - | - | 4.5 |
| Acrylatcopolymer (Acrylates Copolymer) | 2.5 | 2.5 | 3.0 | 3.5 | 3,0 |
| Polyquaternium-10 | 0.1 | 0.1 | 0.05 | 0.25 | 0.2 |
| Guar Hydroxypropyl-Trimonium Chlorid | - | 0.1 | - | - | 0.2 |
| Hydrolysiertes Seidenprotein | - | - | - | - | 0.3 |
| Iminodibersteinsäure | 0.1 | 0.1 | 0.2 | - | - |
| PEG-40 hydriertes Rizinusöl | 0.2 | 0.2 | 0.2 | 0.1 | 0.2 |
| Unispheres (Lactose + Cellulose + Hydroxypropyl Methylcellulose + Cl 77007) | - | 0.2 | - | - | - |
| Natrium Salicylat | - | - | 0.4 | - | |
| Natrium Benzoat | 0.5 | 0.5 | 0.4 | 0.4 | 0.4 |
| Benzophenon-4 | - | - | 0.1 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

| **Mildes Baby Shampoo** | | | | | |
|---|---|---|---|---|---|
| | **1** | **2** | **3** | **4** | **5** |
| Natrium Myristylethersulfat | 4 | 4 | 5 | 5 | 4 |
| Decylglukoside | 4 | 4 | 4 | 4 | 4 |
| Dinatrium PEG-5 Laurylcitrat Sulfosuccinat | 4 | 4 | 3 | 5 | 5 |
| PEG-80 Sorbitan Laurat | 2 | 1 | 1 | - | 0.5 |
| Acrylatcopolymer (Acrylates Copolymer) | 2.5 | 3.2 | 3.5 | 2.0 | 3,0 |
| Polyquaternium-10 | 0.3 | 0.1 | 0.1 | 0.3 | - |
| Guar Hydroxypropyl-Trimonium Chlorid | - | - | 0.1 | 0.2 | 0.2 |
| Perlglanz | - | - | 4 | 2 | 2.5 |
| Trübungsmittel | - | - | - | - | 0.5 |
| Iminodibersteinsäure | - | 0.2 | 0.1 | 0.5 | 0.5 |
| PEG-40 hydriertes Rizinusöl | 0.2 | 0.2 | 0.2 | 0.2 | 0.2 |
| Natrium Salicylat | 0.4 | 0.4 | 0.4 | 0.4 | 0.4 |
| Natrium Benzoat | 0.4 | 0.4 | 0.4 | 0.4 | 0.4 |
| Natriumchlorid | 0.9 | 1.0 | 1.2 | - | - |
| Zitronensäure | q.s. | q.s. | q.s. | q.s. | q.s. |
| Parfüm | q.s. | q.s. | q.s. | q.s. | q.s. |
| Wasser | ad 100 | ad 100 | ad 100 | ad 100 | ad 100 |

| **Antischuppen Shampoo/Mildes Kopfhaut Shampoo** | | | | | | |
|---|---|---|---|---|---|---|
| | **1** | **2** | **3** | **4** | **5** | **6** |
| Natrium Laurylethersulfat | 9 | 9 | 9 | 9 | 10 | - |
| Natrium Myristylethersulfat | - | - | - | - | - | 6 |
| Cocamidopropyl Betain | 4 | 4 | 4 | 4 | 4 | - |
| Dinatrium PEG-5 Laurylcitrat Sulfosuccinat | 3 | 3 | 3 | 3 | - | - |
| Natrium Cocoamphoacetat | - | - | - | - | - | 2.5 |
| Decyl Glucoside | - | - | - | - | - | 2.5 |
| Acrylatcopolymer (Acrylates Copolymer) | 2.5 | 2.8 | 3.0 | 3.0 | 3.5 | 3.5 |
| Polyquaternium-10 | 0.3 | 0.1 | 0.1 | 0.3 | 0.1 | 0.3 |
| Guar Hydroxypropyl-Trimonium Chlorid | - | - | 0.1 | 0.2 | - | - |
| Climbazol | 0.5 | 0.5 | - | 0.5 | 1.0 | - |
| Piroctone Olamin | - | 0.5 | 0.3 | - | 0.5 | - |
| Laureth-9 | - | - | - | - | 2 | 2 |
| Panthenol | - | - | - | - | - | 1 |
| Harnstoff | | | | | | 5 |
| Perlglanz | 1.5 | 3 | 4 | 2 | 2.5 | - |
| Trübungsmittel | - | - | - | - | 0.5 | - |
| Iminodibersteinsäure | 0.1 | 0.2 | 0.1 | 0.5 | 0.5 | - |
| PEG-40 hydriertes Rizinusöl | 0.2 | 0.2 | 0.2 | 0.2 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

| **Kopfhautpeeling-Shampoo** | | | |
|---|---|---|---|
| | **1** | **2** | **3** |
| Natrium Laurylethersulfat | 9 | 9 | 9 |
| Cocamidopropyl Betain | 4 | 4 | 4 |
| Dinatrium PEG-5 Laurylcitrat Sulfosuccinat | 3 | 3 | 3 |
| Acrylatcopolymer (Acrylates Copolymer) | 2.0 | 2.0 | 3.0 |
| Polyquaternium-10 | 0.3 | 0.1 | 0.1 |
| Guar Hydroxypropyl-Trimonium Chlorid | - | - | 0.1 |
| Perlglanz | 1.5 | 3 | 4 |
| Trübungsmittel | - | - | - |
| Iminodibersteinsäure | 0.1 | 0.2 | 0.1 |
| PEG-40 hydriertes Rizinusöl | 0.2 | 0.2 | 0.2 |
| Unispheres (Lactose + Cellulose + Hydroxypropyl Methylcellulose + Cl 77007) | - | - | 0.3 |
| Polyethylenpartikel | 0.1 | 0.2 | 0.1 |
| Natrium Salicylat | 0.4 | 0.4 | 0.4 |
| Natrium Benzoat | 0.4 | 0.4 | 0.4 |
| Natriumchlorid | 0,9 | 1,0 | 1,2 |
| Zitronensäure | q.s. | q.s. | q.s. |
| Parfüm | q.s. | q.s. | q.s. |
| Wasser | ad 100 | ad 100 | ad 100 |

### 13.) Haarstyling Gel

| | **1** | **2** | **3** |
|---|---|---|---|
| PVPNA Copolymer | 5,0 | 6,0 | 7,0 |
| Carbomer | 0,5 | | 0,8 |
| Acrylatcopolymer (Acrylates/C 10-30 Alkyl Acrylate Crosspolymer) | - | 1,0 | - |
| PEG-40 hydriertes Rizinusöl | 0,2 | 0,2 | 0,2 |
| Silikonöl | - | - | 0,1 |
| Glycerin | 3,0 | - | - |
| NaOH | q.s. | q.s. | q.s. |
| Parfum | 0,3 | 0,3 | 0,3 |
| Ethanol | - | - | 10,0 |
| Wasser | ad 100 | ad 100 | ad 100 |

### 14.) Haarkuren

| | **1** | **2** | **3** |
|---|---|---|---|
| Hydroxypropylmethylcellulose | 0,5 | 0,5 | 0,5 |
| Cetrimoniumbromid | 1,0 | - | 0,8 |
| Behentrimoniumchlorid | - | 0,7 | 0,3 |
| Glycerin | 3,0 | 3,0 | 3,0 |
| Cetearylalkohol | 2,5 | 2,5 | 2,5 |
| Glycerylstearat | 2,0 | 2,0 | 2,0 |
| Polyquaternium-10 | 0,1 | - | - |
| Guar Hydroxypropyl Trimonium Chlorid | - | 0,2 | - |
| Panthenol | 0,2 | 0,5 | 0,3 |
| Konservierungsmittel, Parfüm, pH-Ein- | q.s. | q.s. | q.s. |
| stellung und Lösungsvermittler | | | |
| Wasser | ad 100 | ad 100 | ad 100 |

### 15.) Haarspülungen

| | **1** | **2** | **3** |
|---|---|---|---|
| Cetrimoniumchlorid | 1,0 | 0,5 | 0,5 |
| Behentrimoniumchlorid | - | 0,2 | 0,3 |
| Glycerin | 3,0 | 3,0 | 3,0 |
| Hydroxyethylcellulose | 0,2 | 0,2 | 0,2 |
| Polyquaternium-10 | 01 | - | - |
| Guar Hydroxypropyl Trimonium Chlorid | - | 0,2 | - |
| Jojobaöl | 0,2 | 0,3 | 0,1 |
| Konservierungsmittel, Parfüm, pH-Einstellung und Lösungsvermittler | q.s. | q.s. | q.s. |
| Wasser | ad 100 | ad 100 | ad 100 |

### 16.) Leave-on Conditioner

| | **1** | **2** | **3** | **4** |
|---|---|---|---|---|
| Cetylalcohol | 1,5 | 1,8 | 2,0 | - |
| Cetrimoniumchlorid | 0,3 | 0,1 | 0,5 | 0,5 |
| Behentrimoniumchlorid | - | 0,2 | - | - |
| Benzophenone-4 | 0,05 | 0,03 | - | 0,1 |
| PVPNA Copolymer | 0,4 | - | - | - |
| Polyquaternium-37 | - | - | - | 1,0 |
| Polyquaternium-4 | - | - | - | 0,2 |
| Polyquaternium-10 | - | - | 0,5 | - |
| Panthenol | 0,1 | - | 0,2 | 0,1 |
| Hydroxyethylcellulose | - | - | - | 0,3 |
| Acrylates/C10-30 Alkyl Acrylates Cross-polymer | 0,5 | 0,3 | 0,2 | - |
| C12-13 Alkyl Lactate | 2,0 | 1,0 | 1,5 | 1,0 |
| Laureth-4 | - | - | - | 0,5 |
| Aluminium Starch Octenylsuccinate | - | - | - | 1,0 |
| Dicaprylyl Carbonate | - | - | - | 1,0 |
| Konservierungsmittel, Parfüm, pH-Einstellung und Lösungsvermittler | q.s. | q.s. | q.s. | q.s. |
| Wasser | ad 100 | ad 100 | ad 100 | ad 100 |

## Patentansprüche

1. Kosmetik-Produkt, bestehend aus
a. einem Spender für pastöse Produkte mit einem das pastöse Produkt enthaltenden, im Wesentlichen zylindrischen Behälter (1), der bodenseitig einen unter dem Druck der Außenatmosphäre an einer Behälterinnenwand gleitverschieblichen Nachlaufkolben (22) aufweist und an seinem oberen Ende ein zu dem Behälter (1) gleitverschiebliches Kopfstück (3) trägt, welches einen mit dem Behälter (1) kommunizierend verbindbaren Ausgabekanal (32) für das Produkt aufweist und auf eine handbetägigbare Fördereinrichtung mit einer volumenveränderlichen Förderkammer (100) für das Produkt einwirkt, **dadurch gekennzeichnet daß** die Fördereinrichtung ein zu dem Behälter (1) und dem Kopfstück (3) längsverschiebliches Förderelement (5) umfaßt, welches einen in der Förderkammer (100) gleitverschieblichen Förderkolben (51) aufweist, der mit einem Förderschaft (50) verbunden ist, welcher einen Förderkanal (50a) umfänglich umgibt, der eine mit der Förderkammer (100) kommunizierende Förderkanaleinlaßöffnung (53) und eine Förderkanalauslaßöffnung (58) aufweist, die durch eine Verschiebebewegung des Förderelementes (5) relativ zu dem Kopfstück (3) in eine Stellung bringbar ist, in der sich die Förderkanalauslaßöffnung (58) zu dem Ausgabekanal (32) öffnet, und
b. kosmetischen oder dermatologischen Zubereitungen, enthaltend mindestens 0,01 Gew.-% eines oder mehrerer Hydrokolloide.

2. Kosmetik-Produkt nach Anspruch 1, **dadurch gekennzeichnet, daß** die Förderkanalauslaßöffnung (58) an der Umfangsfläche des Förderschaftes (50) ausgespart ist und daß das Kopfstück (3) eine die Förderkanalauslaßöffnung (58) in der Ausgangsstellung (0) der Fördereinrichtung abdeckende Buchse aufweist.

3. Kosmetik-Produkt nach Anspruch 1 oder 2, **dadurch gekennzeichnet, daß** die Buchse als eine die Fördereinrichtung längsverschieblich führende Führungsbuchse (31) ausgebildet ist, die wenigstens eine mit der Umfangsfläche des Förderschaftes (50) zusammenwirkende Führungsfläche aufweist.

4. Kosmetik-Produkt nach einem der vorherigen Ansprüche 2 oder 3, **dadurch gekennzeichnet, daß** an dem Kopfstück (3) und der Fördereinrichtung Mitnehmermittel (34,57) vor gesehen sind, durch welche die Fördereinrichtung nach Handbetätigung bei Rückstellung des Kopfstücks (3) in die Ausgangsstellung (0) mitgenommen wird.

5. Kosmetik-Produkt nach Anspruch 4, **dadurch gekennzeichnet, daß** an der Buchse (31) eine Mitnehmerschulter (57) ausgebildet ist, die mit einem an dem Förderschaft (50) an geformten Mitnehmerkranz (34) zusammenwirkt.

6. Kosmetik-Produkt nach Anspruch 5, **dadurch gekennzeichnet, daß** die Mitnehmerschulter (34) endseitig an der Buchse (31) am Übergang zu dem Ausgabekanal (32) und der Mitnehmerkranz (57) im stirnseitigen Endbereich des Förderschaftes (50) vorgesehen sind.

7. Kosmetik-Produkt nach einem der vorherigen Ansprüche, **dadurch gekennzeichnet, daß** der Förderkolben (51) den Förderschaft (50) zur Ausbildung einer ringförmigen Anlagefläche radial überragt und daß die Führungsbuchse (31) eine stirnseitige Druckfläche (33) aufweist, die in der Ausgangsstellung (0) mit axialem Abstand zu der Anlagefläche angeordnet und durch axiales Verschieben des Kopfstückes (3) in Richtung auf den Behälter (1) an die Anlagefläche anlegbar ist.

8. Kosmetik-Produkt nach einem der vorherigen Ansprüche, **dadurch gekennzeichnet, daß** die Innenwand der Förderkammer (100) durch eine Innenhülse (13) gebildet ist, welche an der kopfstückseitigen Stirnseite des Behälters (1) an der dem Kopfstück (3) zu gewandten Seite an dem Behälter (1) vorgesehen ist.

9. Kosmetik-Produkt nach einem der vorherigen Ansprüche, **gekennzeichnet durch** ein Kopfgegenstück (4), das einen topfförmig auf die Innenhülse (13) gestülpten Haltezylinder (41) sowie einen konzentrisch zu dem Haltezylinder (41) angeordneten, die Gleiterschiebung des Kopfstückes (3) führenden Führungszylinder (42) aufweist.

10. Kosmetik-Produkt nach Anspruch 9, **dadurch gekennzeichnet, daß** das förderkammerseitige Ende des Führungszylinders (42) einen Förderkolbenanschlag für den Förderkolben (51) aufweist.

11. Kosmetik-Produkt nach Anspruch 9 oder 10, **dadurch gekennzeichnet, daß** der Haltezylinder (41) mit einer bodenseitigen Ringschulter (44) versehen ist, die eine Anlagefläche für eine das Kopfstück in der Ausgangsstellung (0) unter Vorspannung haltende Schraubenfeder ausbildet und auf die Stirnseite des Behälters (1) aufgesetzt ist.

12. Kosmetik-Produkt nach einem der Ansprüche 9 bis 11, **dadurch gekennzeichnet, daß** das Kopfgegenstück (4) wenigstens einen Anschlag (46a) zur Begrenzung der axialen Verschiebebewegung des Kopfstücks (3) aufweist und zusammen mit dem Kopfstück (3) als vorgefertigte Spenderkomponente ausgebildet und stirnseitig an dem Behälter (1) befestigt ist.

13. Kosmetik-Produkt nach Anspruch 12, **dadurch gekennzeichnet, daß** die Spenderkomponente mit dem Behälter (1) über an dem Kopfgegenstück (4) und der Stirnseite des Behälters (1) ausgebildete Rastmittel (47 ; 17) verrastet ist.

14. Kosmetik-Produkt nach einem der vorherigen Ansprüche, **dadurch gekennzeichnet, daß** das Kopfstück (3) derart längsverschieblich ist, daß das Kopfstück (3) mittels Handbetätigung von der Ausgangsstellung (0) zunächst um eine erste axiale Wegstrecke (a) zur Anlage an den Förderkolben bei gleichzeitiger Feilegung der Förderkanalauslaßöffnung (58) in dem Ausgabekanal (32) in eine Mittelposition (M) bringbar ist und das Kopfstück (3) danach bei fortschreitender axialer Verschiebung unter Mitnahme des Förderkolbens (51) von der Mittelposition (M) in eine Ausgabe-Endposition (V) bringbar ist, in welcher die Förderkammer (100) durch Verschiebung des Förderkolbens (51) ihr kleinstes Volumen erreicht hat.

15. Kosmetik-Produkt nach einem der vorherigen Ansprüche, **gekennzeichnet durch** ein an dem Kopfteil befestigtes Verschließteil (60), **durch** welches eine Produktabgabeöffnung (39) des Ausgabekanals (32) verschließbar ist.

16. Kosmetik-Produkt nach Anspruch 15, **dadurch gekennzeichnet, daß** die Produktabgabeöffnung (39) ringförmig um einen in dem Ausgabekanal angeordneten Verschlußdorn (32a) ausgebildet ist und daß das Verschließteil (60) eine ringförmig ausgebildete, an den Verschlußdorn dichtend anlegbare Dichtlippe aufweist.

17. Kosmetik-Produkt nach Anspruch 15 oder 16, **dadurch gekennzeichnet, daß** das Verschließteil (60) aus einer weichelastischen Kunststoffmasse, vorzugsweise aus einem thermoplastischen Elastomer gebildet ist.

18. Kosmetik-Produkt nach einem der Ansprüche 15 bis 17, **dadurch gekennzeichnet, daß** das Verschließteil (60) einstückig mit einem zumindest stirnseitig an der Außenseite des Kopfteiles (3) ausgebildeten Überzug (61) ist.

19. Verwendung von mindestens 0,01 Gew.-% eines oder mehrerer Hydrokolloide in kosmetischen oder dermatologischen Zubereitungen zur Verhinderung eines Strukturverlusts der Zubereitungen bei Entnahme aus Spendersystemen, in welchen die Zubereitungen bei der Entnahme einer Scherung ausgesetzt werden.

20. Kosmetik-Produkt nach einem der Ansprüche 1 bis 18 oder Verwendung nach Anspruch 19, **dadurch gekennzeichnet, daß** das oder die Hydrokolloide gewählt werden aus der Gruppe der wasserlöslichen Polysaccharide (Gummis) bzw. deren Derivate.

21. Kosmetik-Produkt nach einem der Ansprüche 1 bis 18 oder Verwendung nach Anspruch 19, **dadurch gekennzeichnet, daß** das oder die Hydrokolloide gewählt werden aus der Gruppe der wasserlöslichen Cellulosen bzw. deren Derivate.

22. Kosmetik-Produkt nach einem der Ansprüche 1 bis 18 oder Verwendung nach Anspruch 19, **dadurch gekennzeichnet, daß** das oder die Hydrokolloide gewählt werden aus der Gruppe der wasserlöslichen Polyacrylsäuren bzw. deren (halb-)synthetische Co- bzw. Crosspolymere.

23. Kosmetik-Produkt nach einem der Ansprüche 1 bis 18 oder Verwendung nach Anspruch 19, **dadurch gekennzeichnet, daß** der Gehalt an dem oder den Hydrokolloiden aus dem Bereich von 0,01 Gew.-% bis 5 Gew.-%, bevorzugt von 0,1 Gew.-% bis 3 Gew.-%, insbesondere von 0,15 Gew.-% bis 1,5 Gew.-% gewählt wird.

24. Kosmetik-Produkt nach einem der Ansprüche 1 bis 18 oder Verwendung nach Anspruch 19, **dadurch gekennzeichnet, daß** das oder die Hydrokolloide gewählt werden aus der Gruppe Polyacrylsäure, Acrylat-Copolymer, Alkyl-Acrylat-Crosspolymer, Ammoniumdimethyltauramide /Vinylformamide Copolymer, Polyacrylamid, Schichtsilikat, Xanthan Gummi und/oder Carrageenan.

25. Kosmetik-Produkt nach einem der Ansprüche 1 bis 18 oder Verwendung nach Anspruch 19, **dadurch gekennzeichnet, daß** eine Hydrokolloidmischung aus mindestens zwei verschiedenen Hydrokolloiden eingesetzt wird.

26. Kosmetik-Produkt oder Verwendung nach Anspruch 25, **dadurch gekennzeichnet, daß** als Hydrokolloidmischung eine Mischung aus:
• Xanthan Gummi und Schichtsilikaten;
• Xanthan Gummi und Polyacrylsäuren;
• Schichtsilikaten und Polyacrylsäuren;
• Cellulosederivaten und Polyacrylsäuren;
• Cellulosederivaten und Schichtsilikaten;
• Ammoniumdimethyltauramide /Vinylformamide Copolymer und Polyacrylat;
• Ammoniumdimethyltauramide /Vinylformamide Copolymer und Polyacrylamid;
• Xanthan Gummi und Polyacrylsäuren und Cellulosederivaten;
• C10-30 Alkyl Acrylate Crosspolymere und Xanthan Gummi;
• Carbomer und Xanthan Gummi;
oder
• 2 verschiedenen Carbomeren
gewählt wird.

27. Verwendung nach Anspruch 19, **dadurch gekennzeichnet, daß** die durch die Scherung bedingte Viskositätsänderung der Zubereitung weniger als 75 % beträgt (Vergleich der Werte vor und nach der Scherung).
